# EUROPEAN PATENT APPLICATION

(11) **EP 2 434 010 A1**
(43) Date of publication of application: **28.03.2012**
(21) Application number: 10777609.8
(22) Date of filing: 04.03.2010
(51) Int. Cl.: C12N 15/09, A23L 1/30, A61K 31/352, A61K 36/75, A61P 3/10, A61P 43/00, C12Q 1/68, G01N 33/15, G01N 33/50

(54) **PROPHYLACTIC AND/OR THERAPEUTIC AGENT FOR METABOLIC SYNDROME**

(30) Priority: 22.05.2009 JP 2009123896
(71) Applicant: Erina Co. Inc., Tokyo 105-0021 (JP)
(72) Inventor: LEE, Young-Sil, Kasugai-shi Aichi 487-8501 (JP); CHA, Byung-Yoon, Kasugai-shi Aichi 487-8501 (JP); WANG, Xiao-Xing, Kasugai-shi Aichi 487-8501 (JP); CHOI, Sun-Sil, Kasugai-shi Aichi 487-8501 (JP); CHOI, Bong-Keun, Kasugai-shi Aichi 487-8501 (JP); YAMAKAWA, Hiroshi, Kasugai-shi Aichi 487-8501 (JP); SAITO, Kiyoto, Kasugai-shi Aichi 487-8501 (JP); YONEZAWA, Takayuki, Kasugai-shi Aichi 487-8501 (JP); TERUYA, Toshiaki, Kasugai-shi Aichi 487-8501 (JP); NAGAI, Kazuo, Kasugai-shi Aichi 487-8501 (JP); WOO, Je-Tae, Kasugai-shi Aichi 487-8501 (JP)
(74) Representative: Held, Stephan
(86) International application number: PCT/JP2010/053588
(87) International publication number: WO 2010/134373

(57) **Abstract**

Disclosed are: a method for screening a substance for treating metabolic syndrome that develops visceral obesity and abnormal lipid metabolism; and a composition for preventing and/or treating the syndrome. The screening method comprises: (1) an extraction step of extracting a total RNA fraction from a skeletal muscle collected from each of non-human mammals that constitute a test substance-administered group to which a predetermined amount of the test substance has been administered for a predetermined period and each of non-human mammals that constitute a test substance-unadministered group to which the test substance has not been administered; (2) a quantification step of quantifying the expression quantity of mRNA contained in the total RNA fraction extracted in the extraction step and encoding a sugar transporter gene by a specific method; and (3) a step of comparing and analyzing the quantity of the test substance ingested, the body weight gain, and the expression quantity of RNA encoding the sugar transporter gene during the predetermined period between the non-human mammals that constitute the test substance-administered group and the non-human mammals that constitute the test substance-unadministered group to thereby determine the effect of the test substance on the metabolic syndrome.

## Description

### Technical Field

The present invention relates to an agent for preventing and/or treating of metabolic syndrome comprising an extract from Mikan-ku citrus as an active ingredient, a pharmaceutical preparation for preventing and/or treating of metabolic syndrome comprising thereof, and functional food for preventing the metabolic syndrome thereof as well as a healthy food thereof.

### Background Art

Obesity causes disease such as hyperglycemia, hypertension, hyperlipidemia and so forth, and increased symptoms becomes common social problems in each country throughout the world.
"Metabolic syndrome (visceral adiposity syndrome)" is defined that it has visceral fat type obesity caused by enlargement of visceral fat tissue to prompt weight gain accompanied by at least two among hyperglycemia, hypertension, and hyperlipidemia; and criterion for the syndrome is published by National Cholesterol Education Program (NCEP).
Diabetes, which is judged based on hyperglycemia as an indicator, is roughly classified into the type 1 and type 2. The type 1 completely lacks secretion of insulin, a hormone to maintain blood sugar level normal, because of mainly inherited factors. Japanese patient number of the type 1 diabetes is 5 to 10 % of whole patient population in ratio. The type 2 diabetes lost sensitivity of insulin posteriori, which is insulin tolerance, and several Japanese cases accompany reduction of insulin secretion. The numbers of the type 2 patient is 90 to 95 % in ratio. The diabetes induced by the metabolic syndrome is type 2 diabetes.

It is known that life style such as overeating places a burden to organs such as kidneys and the like and it contributes to the pathogenesis of diabetes. According to recent studies, enlargement of the adipocytes, the cause of obesity, induces several changes in gene level in the adipocytes to affect the body totally including liver, skeletal muscle and so forth through level change of blood cytokines and other signaling factors to promote the development of diabetes. Pioglitazone is utilized as a very effective hypoglycemic agent for the treatment of diabetes. However, it has side effects (see JP 2003-119148, hereinafter, referred to as Patent Document 1).
The enlargement of the adipocyte has a correlation to hyperlipidemia. As the indicator of hyperlipidemia, total cholesterol level in blood (hereinafter, referred to as "T-CHO" or neutral fat (serum triglyceride, hereinafter, referred to as "TG") have been used. However, at present, LDL cholesterol level in blood is used as the indicator instead of T-CHO. Furthermore, it is known that development of LDL cholesteremia or hyper-triglyceridemia increases a risk for the patient to have cardiovascular disease such as arteriosclerosis and the like. In order to treat such hyperlipidemia, agents such as Statin and Fibrate type ones are used.

On the other hand, there are report that citrus includes substances effective for diabetes or hyperlipidemia as mentioned above. Here, classification of citrus is described in detail in the classification by Tanaka (Tanaka, T., Misunderstanding with regards Citrus classification and nomenclature. Bull. Univ. Osaka Pref. Ser. B, 21, 139-145(1969), hereinafter, referred to as Non-patent Document 1). Note that there is a report that the classification by Tanaka and the kinds and amounts of polymethoxyflavonides included in citrus has a correlation (Non-patent Document 2).
In WO 1999/056768 A2 (hereinafter, referred to as "[Patent Document 2"), it is shown that a method for evaluating a material by using change of expression amount of a gene expressed in fatty acid tissue or in an adipocyte (fat cell) as in the indicia. For example, it is reported that GDF-8 inhibitor promotes GLUT4 in the cultured adipocyte, and the inhibitor is useful for treating diabetes.

Alternatively, it is reported for effect of polymethoxyflavonides (hereinafter, referred to as "PMF") to following matters are reported. In WO 2007/024982 A2 (hereinafter, referred to as Patent Document 3), there is reported that mice given high fat diet supplemented with PMF fraction extracted from orange (Citrus sinensis) has both of less weight gain and average fat weight compared to those given the high fat diet only.
In JP 2001-240539 (hereinafter, referred to as Patent Document 4), when nobiletin, one of PMF, or condensed juice of flat lemon (Shiikuwasha) is orally administered to mice with spontaneous diabetes, blood glucose increase in them are suppressed compared to the mice not given them.
In WO 2002/087567 A2 (hereinafter, referred to as Patent Document 5), when the guinea pig are given fructose only or fructose with an edible tangeretin or PMF composition from citrus, total cholesterol level in blood became lower in those with PMF.

### Prior Art

### [Patent Document]

[Patent Document 1] JP2003-119148A
[Patent Document 2] Intenational PublicationWO 1999/056768 A2
[Patent Document 3] Intenational PublicationWO 2007/024982 A2
[Patent Document 4] JP2001-240539A
[Patent Document 5] Intenational PublicationWO 2002/087567 A2

### [Non-Patent Document]

[Non-Patent Document 6] Tanaka, T., Misunderstanding with regards Citrus classification and nomenclature. Bull. Univ. Osaka Pref. Ser. B, 21, 139-145 (1969)
[Non-Patent Document 7] Nogata, Y., Sakamoto, K., Shiratsuchi, H., Ishii, T., Yano M., and Ohta, H., Flavonoid Composition of Fruit Tissues of Citrus Species, Biosci. Biotechnol. Biochem., 70, 178-192 (2006)

### Summary of the Invention

### Problem to be Solved by the Invention

As described above, since the metabolic syndrome is induced by visceral fat type obesity, it is essential to improve the degree of obesity, namely, reduce fat tissue in size to lead more effective treatments. However, at present, effective anti-obesity agent has neither been found nor approved, diet therapy and exercise therapy are combined to use.
Above-mentioned Pioglitazone includes discrepancies that it enhances growth of the adipocyte. Therefore, if the patient takes it for a prolonged period, there are problems to gain weight or body fat and to have an impaired response to hypoglycemic action. Furthermore, Statin type or Fibrate type agents have the side effect that causes rhabdomyolysis or increases of secretion of fatty acids from liver.

For the treatment of the metabolic syndrome, diet restriction and appropriate exercise are important; because the following cycle, the blood glucose taken in a muscle but unused is stored in adipose tissues to enlarge the adipocytes, which secretes neutral fat that causes complications of circulatory system should be stemmed. Namely, it is necessary for the effective treatment to reduce excess ingestion of glucose source and fully consume ingested glucose. Here, reduction of glucose source ingestion, namely food restriction, should be carefully performed not so as to cause hypoglycemia or osteoporosis. Alternatively, accelerated blood glucose intake into a muscle and metabolism in it enhance blood sugar consumption. By this, the blood glucose amount incorporated into enlarged adipocyte decreases, and it reduces adipocyte enlargement to inhibit neural fat secretion and the like.
Furthermore, as mentioned above, the metabolic syndrome accompanies plural pathological conditions so that it causes limitation of supportive therapy that is defined that an agent is administrated depending on the pathological condition. Therefore, there are strong social needs for (A) a substance that ameliorates obesity and hyperglycemia, but does not inhibit the improvement of the hyperlipidemia, or (B) a screening of the substance that ameliorates the obesity and the hyperlipidemia but does not inhibit the improvement of the hyperglycemia.

In order to prophylaxis and/or treatment of the metabolic syndrome, it is mostly preferable that the substance being capable of simultaneously ameliorating the above-mentioned three pathological conditions such as visceral fat type obesity, the hyperglycemia, and the hyperlipidemia, with no or weak side effects such as hypertension and so forth, is found to be provided as the agents. However, even if the agent is not capable of simultaneously ameliorate the above-mentioned three pathological conditions, there is no problem unless either the hyperglycemia or the hyperlipidemia became worse, if it is capable of ameliorating the visceral fat type obesity and either one of hyperglycemia or hyperlipidemia.
Furthermore, if the substance is highly safe, it can be provided as a pharmaceutical preparation and also a functional food or healthy food. By this, it is expected that the agent has an effect to prevent the generation of the patient with the metabolic syndrome.
Accordingly, there are strong social needs to provide such a substance.

### [Means for Solving the Problem]

The present invention is completed under the above-mentioned situation. That is, the present inventors pursued the development of a screening method of components which are effective for the treatment of the metabolic syndrome based on complicated mechanism which associate visceral fat type obesity, hyperglycemia, and hyperlipidemia; considering safety and side effects. As a result, they found that it is possible to screen the components effective to the metabolic syndrome treatment by determining mRNA expression level of glucose transporter gene in the skeletal muscle tissue or the skeletal muscle cells. They found that fractions of the citrus extracts, which are characterized with the glucose transporter gene, have remarkable unknown effects by the screening to complete the present invention.

The first aspect of the present invention is a screening method for treatment of the metabolic syndrome showing visceral fat type obesity, hyperglycemia, and hyperlipidemia having the following steps (1) to (3): (1) extracting total mRNA fraction from skeletal muscle excised from an non-human mammal animal selected from the group consisting of a test substance administration group and a test substance non-administration group, wherein the animal of the test substance administration group is given the test substance at an predetermined amount in predetermined term and that of the test substance non-administration group is not given the test substance; (2) determining an expression amount of mRNA that codes glucose transporter gene included in the respective total RNA fraction extracted in the extraction step by using a predetermined method; and (3) deciding an effect to the metabolic syndrome by using comparison analysis for body weight gain amount and ingestion amount, and the expression amount of RNA of the glucose transporter gene in the predetermined period between the test substance administration group and the test substance non-administration group. The glucose transporter gene to be determined in the above-mentioned step (2) is preferably mouse GLUT4 or homolog thereof.

In the screening method, the predetermined method preferably comprises the steps of synthesizing cDNA from RNA that codes the glucose transporter gene by using a reverse transcriptase to amplify the cDNA as a template with specific primers for the glucose transporter gene, determining the amplified products by using a detection method that matches to a regent selected from the group consisting of fluorescently-labeled nucleic-acid, isotope-labeled nucleic acid, and nucleic acid staining; and determining the expression amount of RNA that codes the glucose transporter gene.
Further, the step of determining the expression amount of RNA that codes glucose transporter gene may be substituted with a step determining an expression amount of glucose transporter protein being coded the gene by using the predetermined method. In such a case, in the comparison analysis, it is preferable to perform comparative analysis between the groups by using the expression amounts of the protein instead of the expression amounts of RNA.

The predetermined method for determining the expression amount of the protein preferably comprises the steps of determining that of the glucose transporter by using a method selected from the group consisting of Western blotting, ELISA, and immunostaining; and deciding the expression amount. Alternatively, the method relying on the RNA expression level also may be used with that relying on the protein expression level and both of them may be utilized for the comparison analysis. Furthermore, the test substances include a compound and a composition.
The second aspect of the present invention is a reagent kit for performing the screening method. The reagent kit preferably comprises the reverse transcriptase, an enzyme amplifying DNA by using cDNA as a template, and a glucose transporter gene specific primer. The reagent kit preferably comprises nucleic acid stain agent.

The third aspect of the present invention is a composition for treating the metabolic syndrome accompanying visceral fat type obesity, hyperglycemia and hyperlipidemia in precritical stage of diabetes, comprising a peel extract fraction of Mikan-ku (Acrumen (VII) section) citrus which contains 70 to 100 wt % of nobiletin in the dry weight equivalent, which is administering at the dose of 50 to 200 mg/kg per day in the dry weight equivalent per os and it has the following functions or characterizations (1) to (4): (1) A function to reduce a body weight gain without any affection for ingestion amount; (2) a function to promote expression of protein of a mouse glucose transporter gene, GLUT 4 and a homolog thereof, in a skeletal muscle tissue or a skeletal muscle cell; (3) a function to promote to shrink an enlarged white adipose tissue located periphery of viscera; and (4) a function to decrease blood glucose level without any affection to blood triglyceride.
In the composition, the Mikan-ku (Acrumen (VII) section) citrus is preferably Shiikuwasha: Citrus depressa, nobiletin is preferably contained in 95 to 100 wt % in the dry weight, the dose is preferably 10 to 100 mg/kg per day; and the function or the characterization preferably further comprises (5) a function to promote to shrink an adipose cell of the white adipose tissue located in a periphery of viscera; and (6) a function to ameliorate glucose tolerance.

The fourth aspect of the present invention is a composition for prophylaxis and/or treatment of the metabolic syndrome accompanying visceral fat type obesity, hyperglycemia and hyperlipidemia, comprising peel extract fraction of Mikan-ku (Acrumen (VII) section) citrus that includes 45 to 55 wt % of nobiletin and 45 to 55 % of tangeretin, and administering a dose of 0.5 to 8.0 g/body/day of the fraction in the dry weight equivalent of the peel fraction having following functions (1) to (4): (1) a function to reduce a body weight gain without any affection for ingestion amount; (2) a function to promote expression of mRNA or protein of a mouse glucose transporter gene, GLUT 4 and a homolog thereof, in a skeletal muscle tissue or a skeletal muscle cell; (3) a function to promote to shrink an enlarged white adipose tissue located periphery of viscera; and (4) a function to decrease blood triglyceride level without any affection to blood glucose.
In the composition, the Mikan-ku (Acrumen (VII) section) citrus is preferably Shiikuwasha: Citrus depressa, the dose is preferably 1.0 to 6.0 g/body/day. Furthermore, as the function or the characterization, it further comprises that to promote to shrink an adipose cell of the white adipose tissue located in a periphery of viscera; and (6) a function to ameliorate leptin tolerance.

Furthermore, the fifth aspect of the present invention is a functional food containing the composition for treating the metabolic syndrome. The sixth aspect of the present invention is a healthy food comprising the composition for treating the metabolic syndrome. The seventh aspect of the present invention is a pharmaceutical preparation comprising the composition for treating the metabolic syndrome.

### Effect of the Invention

According to the screening method of the present invention, a material having following functions or characterization may be chosen; (1) the function to reduce the body weight gain without any affection for ingestion amount; (2) the function to promote expression of protein of glucose transporter gene such as mouse GLUT 4 and others in a skeletal muscle tissue or a skeletal muscle cell.
According to the composition comprising nobiletin, the pharmaceutical preparation, functional food or healthy food, which are used for prophylaxis and/or treatment of the metabolic syndrome having anti-obesity effect and hyperglycemia treatment effect without preventing hyperlipidemia amelioration, may be provided. According to the composition comprising nobiletin and tangeretin, the pharmaceutical preparation, functional food or healthy food, which are used for prophylaxis and/or treatment of the metabolic syndrome having anti-obesity effect and hyperlipidemia treatment effect without preventing hyperglycemia amelioration, may be provided.
According to the method by administrating the present pharmaceutical preparations in a desirable way, the metabolic syndrome can be prevented and/or be treated. Also, by ingesting the functional food or healthy food of the present invention, the metabolic syndrome can be prevented and/or treated.

### Brief Description of the Drawings

[Fig. 1A] This is a graph showing a result of HPLC analysis of Shiikuwasha peel extract. A vertical axis of the graph shows potential change of UV detector as detecting voltage (mV).
[Fig. 1B] This is the graph showing a result of HPLC analysis of citrus unshiu peel extract. The vertical axis of the graph shows potential change of UV detector as detecting voltage (mV).
[Fig. 2A] This is the graph showing gained weight of mice given a low fat diet (LFD), a high fat diet (HFD), a Shiikuwasha extract supplemented high fat diet (HFD + 1SPE), and a high concentration Shiikuwasha extract supplemented high fat diet (HFD + 1.5SPE) in the example 3.
[Fig. 2B] This is the graph showing ingestion weight by the mice given the LFD, the HFD, the HFD + 1SPE, the HFD + 1.5SPE in the example 3.
[Fig. 2C] This is the graph showing weight of liver, kidney and white adipose tissue of the mice given the LFD, the HFD, the HFD + 1SPE, the HFD + 1.5SPE in the example 3.
[Fig. 3A] This is the graph showing levels of neutral fat (triglyceride), total cholesterol, glucose in blood of the mice given the LFD, the HFD, the HFD + 1SPE, the HFD + 1.5SPE in the example 3.
[Fig. 3B] This is the graph showing level of leptin in blood of the mice given the LFD, the HFD, the HFD + 1SPE, the HFD + 1.5SPE in the example 3.
[Fig. 3C] This is the graph showing relative expression levels of lipid biosynthesis related genes in the white adipose tissue of the mice given the LFD, the HFD, the HFD + 1SPE, the HFD + 1.5SPE (as that of LFD = 1) in the example 3.

[Fig. 4A] This is a photograph observing the adipose cells of the white adipose tissue of the mice given the LFD in the example 3.
[Fig. 4B] This is the photograph observing the adipose cells of the white adipose tissue of the mice given the HFD in the example 3.
[Fig. 4C] This is the photograph observing the adipose cells of the white adipose tissue of the mice given the HFD + 1SPE in the example 3.
[Fig. 4D] This is the photograph observing the adipose cells of the white adipose tissue of the mice given the HFD + 1.5 SPE in the example 3.
[Fig. 5A] This is the graph showing the blood glucose level when any one of a sole solvent (vehicle), nobiletin (No200), or Pioglitazone (P30) was given to the diabetes model mice.
[Fig. 5B] This is the graph showing glucose tolerance as the results of oral glucose tolerance test (OGTT) when the vehicle, No200, or P30 was given to the diabetes model mice.

[Fig. 6A] This is the graph showing the mRNA expression level of glucose transporter gene, GLUT4, in the white adipose tissue when the vehicle, No200, or P30 was given to the diabetes model mice.
[Fig. 6B] This is a combination of the photograph of electrophoresis pattern showing the GLUT 4 protein expression level and the graph showing them, when the vehicle, nobiletin (No200), or Pioglitazone (P30) was given to the diabetes model mice.
[Fig. 6C] This is a combination of the photograph of electrophoresis pattern showing the GLUT 4 protein expression level and the graph showing them in the skeletal muscle, when the vehicle, No200, or P30 was given to the diabetes model mice.
[Fig. 7A] This is the graph showing mRNA expression level of the gluconeogenesis-related gene, PEPCK, in a liver tissue, when the vehicle, No200, or P30 was given to the diabetes model mice.
[Fig. 7B] This is the graph showing mRNA expression level of the gluconeogenesis-related gene, G6Pase, in the liver tissue, when the vehicle, No200, or P30 was given to the diabetes model mice.

[Fig. 8A] This is the graph showing the blood adiponectin level, when the vehicle, No200, or P30 was given to the diabetes model mice.
[Fig. 8B] This is the graph showing mRNA of the adiponectin gene expression level, when the vehicle, No200, or P30 was given to the diabetes model mice.
[Fig. 9A] This is the graph showing the mRNA expression level of an adipokine, TNF-α, when the vehicle, No200, or P30 was given to the diabetes model mice.
[Fig. 9B] This is the graph showing the mRNA expression level of the adipokine gene, MCP-1, when the vehicle, No200, or P30 was given to the diabetes model mice.
[Fig. 9C] This is the graph showing the mRNA expression level of the adipokine gene, IL-6, when the vehicle, No200, or P30 was given to the diabetes model mice.

[Fig. 10A] This is the graph showing the mRNA expression level of an adipocyte-specific transcription factor gene, PPARγ, when the vehicle, nobiletin (No200), or Pioglitazone (P30) was given to the diabetes model mice.
[Fig. 10B] This is the graph showing the mRNA level of a lipid biosynthesis-related gene, aP2, when the vehicle, No200, or P30 was given to the diabetes model mice.
[Fig. 10C] This is the graph showing the mRNA level of a lipid accumulation control-related gene, LPL, when the vehicle, No200, or P30 was given to the diabetes model mice.
[Fig. 10D] This is the graph showing the mRNA level of the lipid accumulation control-related gene, Perilipin, when the vehicle, No200, orP30 was given to the diabetes model mice.

[Fig. 11A] This is the combination of the photograph of electrophoresis pattern showing the mRNA expression levels of the adipocyte-specific transcription factor, PPARγ gene, and the glucose transporter, GLUT4 gene, in the white adipose tissue of the LFD ingestion group (LFD; comparison example without obesity-induction) to which the low fat diet + vehicle were administrated, HFD ingestion group to which the high fat diet (HFD) + the vehicle were administrated to which the high fat diet + tangeretin were administrated (HFD + T200) in the example 6.
[Fig. 11B] This is the combination of the photograph of electrophoresis pattern showing the mRNA expression levels of the glucose transporter, GLUT4 gene, in the skeletal muscle of the above-mentioned LFD, HFD, HFD + T200 after the obesity induction in the example 6.
[Fig. 12A] This is the graph showing the obesity-induction schedule by using the high fat diet (HFD) and the administration schedule of nobiletin or tangeretin in the examples 6 and 7.
[Fig. 12B] This is the graph showing the weight gain of the LFD ingestion group to which low fat diets and the vehicle were administrated (LFD; the comparison example without the obesity-induction), the HFD ingestion group to which high fat diet + the vehicle were administrated after the obesity induction by using the high fat diet (HFD), the 10No administration group to which the high fat diet + nobiletin were administrated (HFD + 10No), and 10No administration group to which the high fat diet + the high concentration of nobiletin was administrated (HFD + 100No) for 5 weeks in the example 7.
[Fig. 12C] This is the graph showing an average ingestion amount of LFD, HFD, HFD + 10No, and HFD + 100No for 5 weeks in the example 7.

[Fig. 13A] This is the graph showing the weight of liver, kidney and white adipocyte tissue of LFD, HFD, HFD + 10No, and HFD + 100No in the example 7.
[Fig. 13B] This is the graph showing the levels of neutral fat (triglyceride), total cholesterol, and glucose in blood of LFD, HFD, HFD + 10No, HFD + 100No in the example 7.
[Fig. 14A] This is the graph showing the glucose tolerance of LFD, HFD, HFD + 10No, HFD + 100No during the oral glucose tolerance test (OGTT) in the example 7.
[Fig. 14B] This is the graph showing value of integral in time of the blood glucose level curve of LFD, HFD, HFD + 10No , HFD + 100No during the oral glucose tolerance test (OGTT) in the example 7.

[Fig. 15A] This is the photograph showing the adipocytes of white [Fig. 15A] This is the photograph showing the adipocytes of white adipose tissue of the LFD in the example 7.
[Fig. 15B] This is the photograph showing the adipocytes of white adipose tissue of the HFD in the example 7.
[Fig. 15C] This is the photograph showing the adipocytes of white adipose tissue of HFD + 10No in the example 7.
[Fig. 15D] This is the photograph showing the adipocytes of white adipose tissue of HFD + 100No in the example 7.

### Embodiment for Carrying Out the Invention

In below, the present invention is explained in detail.
In the present specification, unless otherwise noted, the metabolic syndrome is defined as that presenting with visceral fat type obesity, hyperglycemia, and hyperlipidemia. Furthermore, in the present invention, the anti-obesity effect includes an effect to shrink the enlarged visceral fat.
As a test substance, any substances may be used. The test substance is preferably orally administrated to an animal as a food mixed with it, dissolved or suspended in a suitable solvent solution. Furthermore, the test substance may be administrated percutaneously to the animal, if it is lipophilic.

The non-mammal animal to be tested for the screening method of the present invention is preferably Rodentia, more preferablly, mouse for experiment (Mouse musculus; hereinafter, it is referred to as "mouse".). A plurality of group is preferably composed of mice; wherein a test sample administration group to which the test sample is given at predetermined dose during predetermined period and test sample non-administration group to which no test sample is given, or other groups for which predetermined conditions such as the dose or food are varied.
In the screening method of the present invention, it is preferable to measure body weight as an index of obesity. It is more preferable to measure degree of the visceral fat type obesity to be excised by dissection to measure the weight of white adipose tissue of viscera. Further, it is preferable to measure the degree of the adipose cell enlargement by observing the adipose tissue. Alternatively, as the method without the dissection, body fat percentage may be measured by using a method for measuring electrical resistance of the test animals or density thereof. As the method for measuring food intake, it is preferable to weigh reduced amount of the food given to the mice in a cage, because it is convenient.

As the glucose transporter-related genes to be used in the screening method of the present invention, there are preferably mentioned such as GLUT4, GLUT1, GLUT2, AMPK and the like. If the substance for treating the metabolic syndrome controls expressions of such genes in skeletal muscle, insulin resistance, impaired glucose tolerance, or hyperglycemia may be ameliorated by controlling the blood glucose intake efficiency into the skeletal muscle tissue. Furthermore, reducing the glucose intake amount into the adipose cells inhibits the enlargement of the adipose cells and the adipose tissues, and promotes to shrink an enlarged adipocytes and the adipose tissue. Normalizing a lipid secretion from the shrunk adipose cells and the adipose tissue makes ameliorate hyperlipidemia. Furthermore, if the substance for treating the metabolic syndrome is promoted the glucose transporter-related genes such as GLUT4, AMPK and the like in the skeletal muscle, it is expected for the metabolic syndrome patient who needs exercise therapy to provide auxiliary effect. In hyperglycemia, the acceleration of the expression of these genes provides to prevent progress into more severe diabetes by ameliorating insulin resistance. Among these genes for the screening, GLUT4 in the skeletal muscle is especially preferable, because it shows that the substance has high possibility to ameliorate symptoms of hyperglycemia and hyperlipidemia by the substance.

The measurement of the gene expression of the present invention may be performed by determining the mRNA or protein expression. These measurements may be performed by using the known methods. The determination of mRNA preferably comprises the following steps of:
(1) extracting fractions containing mRNA from skeletal muscle sections, or skeletal muscle cells;
(2) synthesizing cDNA from RNA that codes glucose transporter by using a reverse transcriptase;
(3) amplifying the cDNA by using the transporter gene specific primers;
(4) determining the amplified products by using the predetermined regent and the detecting method which matches to it; and
(5) deciding the expression level of RNA that codes the glucose transporter gene.
The reagents are preferably selected from the group consisting of fluorescently-labeled nucleic-acid, isotope-labeled nucleic acid, and nucleic acid staining. In view of screening efficiency, the nucleic acid staining is more preferable. As the nucleic acid staining, SYBR (registered trademark) Green is preferable in view of detection efficiency. As the detection method, the real time PCR is preferable in view if precision. As the method for detecting the expression level, the comparison Ct method by using glyceraldehyde 3-phosphate dehydrogenase (GAPDH) as a standard reference is preferable.
Furthermore, the method for protein expression level preferably comprises a step for extracting total protein fraction from the skeletal muscle section or the skeletal muscle cell; determining the expression level of the glucose transporter protein in the protein faction by using Western blotting, ELISA, and other known method. Alternatively, the expression level of the protein may be measured by using the immunostaining of the skeletal muscle tissue or skeletal muscle cells.

The decision of the effect from the test substance of the present invention to the metabolic syndrome is preferably performed based on the following conditions.
Namely, under the above-mentioned predetermined conditions, when it is compared to the test substance non-administration group, it is preferably meet the conditions: (Condition 1) it reduced weight gain or gained amount of the weight; (Condition 2) it does not accompany remarkable increase or decrease of food ingestion; (Condition 3) it enhances the expression of mRNA or the protein of the glucose transporter gene in the test substance administration group. In the (Condition 1), the substance having obesity inhibition effect is chosen, in the (Condition 2), the substance having the food ingestion decrease, and that having the food ingestion increase that leads enhancement of the obesity, both of which are indicia of the side effect, are eliminated. Then, in the (Condition 3), the substance to enhancing the glucose transporter gene expression in the skeletal muscle for promising amelioration of hyperglycemia or hyperlipidemia is chosen.

In order to obtain the present composition for prophylaxis and/or treatment of the metabolic syndrome, it is preferable from Mikan-ku citrus peel because of extraction efficiency. Among the Mikan-ku citrus, Keraji (Keraji; Citrus Keraji; hereinafter, it referred to as the same), Ponkan (Ponkan; C. reticulata), Dancy tangerine (Dancy tangerine; C. tangerine), Jimikan (Jimikan; C. succosa), Shikaikan (Shikaikan; C. suhuiensis), Tachibana (Tachibana; C. tachibana), Kobenimikan (Kobenimikan; C. erythrosa), Kishumikan (Kishumikan; C. kinokuni), Flat lemon (Shiikuwasha; C. depressa), Koji (Koji; C. leiocarpa) and the like are more preferable because of the contents of the composition.
From the view point of working efficiency for obtaining peel from the fruits and extraction efficiency of the composition, it is more preferable to extract from Shiikuwasha (Shiikuwasha; C. depressa). The peel can be dried under the sunshine or by using a mechanical drier. However, drying under the sunshine is preferable for decreasing energy consumption for drying and preventing decomposition of the components.

As the composition of the present invention for prophylaxis and/or treatment of the metabolic syndrome, the extract, which is obtained from the dry peel of the Mikan-ku citrus by MeOH extraction and the like, may be preferably used. The extract includes nobiletin shown in the following formula (I) and/or tangeretin shown in the following formula (II) in a predetermined ratio. Further, a group of the compound of which backbone moiety is common with nobiletin and tangeretin, but numbers and sites of methoxy group are different is generally referred to as polymethoxyflavonides (PMF) so that the same name is used in the specification.

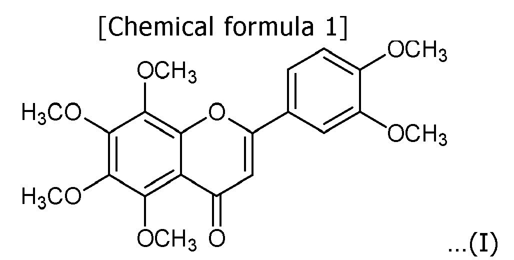

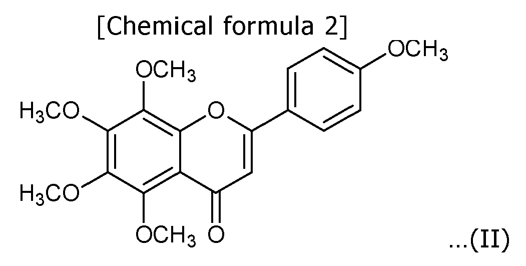

In below, it is shown one example of the method for extracting the fraction including PMF from the above-mentioned plant materials to partially purify to obtain the fraction including nobiletin and tangeretin in the predetermined ratio, and the method for purifying nobiletin or tangeretin.
The peel from the citrus fruit was dried under sunshine for 3 days to prepare the dry peel. To the predetermined amount of dry peel, 5 times volume of a solvent is added, and then it was performed to immersion extraction at a predetermined temperature in a predetermined term to obtain the extract. Here, as an extraction solvent, there are mentioned, for example, MeOH, EtOH, acetone, ethylacetate, and water-MeOH mixture, water-EtOH mixture and the like in various mixture ratios.
Obtained crude extract is filtrated to separate solid content, and then whole amount is concentrated to obtain condensate. Suitable amount of mixture of aqueous phase/organic phase is added to the condensate to perform liquid-liquid partition extraction. The partition extraction is repeated a plurality of times by using the mixture with different mixture ratio. The organic phase obtained by using the partitioned extraction is concentrated to be subjected to an open column. It is eluted by using a step gradient method, stepwisely the solvent concentration is increased, the desirable fractions is used as peel extract (hereinafter, it is referred to as "SPE".).

Next, a part of the fraction is condensed, and then its solute concentration is prepared to be subjected to a preparative column chromatography to partial purification. The SPE is subjected to further column chromatography to determine amounts of nobiletin and/or tangeretin included in the fraction. The fraction includes the desirable content of nobiletin, or both of nobiletin and tangeretin is used as the composition for treatment explained in below.
Concretely, 30 to 50 L of MeOH is added to 6 to 10 kg of the dry peel of Mikan-ku citrus such as Shiikuwasha, citrus unshiu (Satsuma; C. unshiu) and the like to perform the extraction from 7 to 21 days at 2 to 6 °C to obtain the extract. The extract is filtrated to separate the solid content, and then whole filtrate is condensed by using a flash evaporator. Next, 4 L of water/ ethylacetate (1/1) is added to the condensate to perform the partitioned extraction. Four L of 90% MeOH/n- hexane (1/1) is added to the obtained ethylacetate phase, liquid-liquid distribution extraction is repeated.

Subsequently, a part of the 90% MeOH phase is concentrated to be applied, for example, on ODS silica gel column (Cosmosil 75C₁₈-OPN, 50 mmϕ × 500 mm), to perform step gradient elution to increase MeOH concentration by 20% to obtain 60% to 100% MeOH fractions. The fractions were evaporated by using a large size rotary evaporator to use them as SPE for the present composition of prophylaxis and/or treatment for the metabolic syndrome.
Next, for example, among the above-mentioned fractions, a part of the 80% MeOH fraction is concentrated, and MeOH is added into the concentrate to prepare the solution including 900 mg/mL of solute. Then, it is subjected to a preparative column chromatography. For example, desirable fractions are obtained by fractionation, nobiletin and/or tangeretin in each fraction are determined.

Preparative conditions:
Column: C₁₈-AR-II column (NACALAI TESQUE, INC.)
Elution solvent: 70% MeOH/water
Solute concentration: 900 mg/mL
Injection volume: 3 mL
Fraction volume: 10 mL
Flow rate: 5 mL/minute
Detection wave length: UV 215 nm
Range: 20 mV
Retention time of reference (Retention Time) standard of nobiletin and tangeretin and that of the peaks of the fraction are analyzed based on a chromatogram, and determine nobiletin and tangeretin. In order to obtain the composition for prophylaxis and/or treatment of the metabolic syndrome of the present invention, it is used after drying by the evaporator and the like. Thermal drying method is not preferable, because it may cause thermal degradation of the components.
Note that the composition containing nobiletin and/or tangeretin used in the composition for prophylaxis and/or treatment for the metabolic syndrome of the present invention may be obtained by using known methods or the equivalent method, or by purchasing commercially available things.

The composition for prophylaxis and/or treatment for the metabolic syndrome of the present invention preferably control the metabolic syndrome-related genes. The metabolic syndrome-related genes are defined as the gene selected from the group consisting of lipid biosynthesis-related genes, glucose transporter-related genes, gluconeogenesis-related genes, adipokine-related genes, adipocyte-specific transcription factor, and lipid accumulation control-related gene. Here, in the specification, the term, "control", includes the case for increasing, decreasing and maintaining the above-mentioned gene expression.
As the lipid biosynthesis-related genes, there are mentioned genes such as aP2, SREBP1c, SCD1, FAS, ACC1, FATP, DGAT1, AGPAT, GPAT and the like. Among them, it is preferable to control the expression of the gene selected from the group consisting of aP2, REBP1c, SCD1, FAS, ACC1, FATP, and DGAT to inhibit the enlargement of the adipose cells through the inhibition of the lipid biosynthesis, as a result, it inhibits the enlargement of the adipose tissue and improves the insulin resistance, impaired glucose resistance, hyperglycemia, or blood hyperlipidemia.

The he composition for prophylaxis and/or treatment for the metabolic syndrome of the present invention more preferably controls the expression of the glucose transporter genes, mouse GLUT4 and homolog thereof, in the adipose tissue; because it regulates the intake of blood glucose into the tissue to suitably control the status of adipose tissue, and blood glucose level or blood neutral fat.

As the gluconeogenesis-related genes, there are mentioned those such as PEPCK, G6Pase, fuructose-1,6-bisphosphatase, pyruvate carboxylase and the like. The gene which enables to inhibit the expression of PEPCK or G6Pase in the liver is preferable, because it enables to ameliorate the insulin resistance in the liver by inhibiting gluconeogenesis activity.
As the adipokine-related genes, there are mentioned so-called adipokines such as TNFα, MCP-1, IL-6, PAI-1, RBP4, Resistin, Adipsin and the like. Because, the bad adipokine production is inhibited by inhibiting the expression of at least one gene selected from the group consisting of TNFα, MCP-1, IL-6, and PAI-1, thereby these bad adipokine secretion is inhibited. As a result, the insulin resistance in the liver is ameliorated.

As the adipocyte-specific transcription factor gene, there are mentioned those such as PPARγ, PDE3B and the like. Among them, enhanced PPARγ expression leads improvement of PPARγ functions; as a result, adiponectin gene expression is enhanced. As a result, adiponectin production and secretion are enhanced.
At this time, selective inhibition of the lipid biosynthesis-related gene expression by PPARγ leads the enlargement of the adipose cell inhibition, and the high advantage in the amelioration of insulin resistance.
As the lipid accumulation control-related gene, there are mentioned those such as LPL, Perilipin, PPARσ and the like. Among them, LPL expression or Perilipin expression is preferably enhanced. By this, the accumulation of fat is inhibited, it leads the inhibition of the adipose cell enlargement; as a result, and it inhibits the adipose tissue enlargement. Then, insulin resistance, impaired glucose tolerance, hyperglycemia, or blood hyperlipidemia are ameliorated.

The composition for prophylaxis and/or treatment for the metabolic syndrome of the this aspect of the present invention is preferably that containing 70 to 100 wt % of nobiletin to the dry weight equivalent of the purified substance; the dose per day is preferably 5 to 200 mg/kg body weight in nobiletin equivalent per os. It is more preferable that the nobiletin content in the composition is 95 to 100 wt %, and the dose per day is 10 to 150 mg/kg of body weight in nobiletin equivalent; it is more preferable 100 mg/kg of body weight.
In the above-mentioned matters, the composition containing nobiletin in the above-mentioned range has the functions such as (1) to inhibit a body weight gain without any affection for ingestion amount; (2) to enhance protein expression of a mouse glucose transporter gene, GLUT 4 and a homolog thereof, in the skeletal muscle tissue or the skeletal muscle cell; (3) to promote to shrink the enlarged white adipose tissue located periphery of viscera; (4) to decrease blood glucose level without any affection to blood triglyceride; (5) to shrink an enlarged white adipose tissue located periphery of viscera; and (6) to ameliorate glucose tolerance.

The fourth aspect of the composition for prophylaxis and/or treatment for the metabolic syndrome of the present invention is preferably the SPE which contains 40 to 60 wt % of nobiletin and 40 to 60 % of tangeretin to the dry weight of the composition. More preferably, it is the SPE which contains 45 to 55 wt % of nobiletin and 45 to 55% of tangeretin.
The dosage amount to human of the fourth aspect of the present invention is preferably as follows. In general, since about 50 times higher amount of that of the mouse is for human dose, the dosage amount per day of the composition for an adult is preferably 1 to 4 g/body per os, more preferably 2.0 to 3.4 g/body, further preferably 3.2 to 3.4 g/body. Furthermore, it is preferable to adjust the dosage in the range of about 1/2 to 2 times of that depending on the body weight of the patient.
In the above-mentioned matters, the composition containing the SPE has the functions such as (1) to inhibit a body weight gain without any affection for ingestion amount; (2) enhance expression of protein of a mouse glucose transporter gene, GLUT 4 and a homolog thereof, in a skeletal muscle tissue or a skeletal muscle cell; (3) to promote to shrink an enlarged white adipose tissue located periphery of viscera; (4) to decrease blood triglyceride level without any affection to blood glucose; (5) to promote to shrink an enlarged white adipose tissue located periphery of viscera; and (6) to ameliorate leptin tolerance.
Among the functions shown by nobiletin, SPEs which has nobiletin and tangeretin in the above-mentioned range are different at having effects to reduce blood triglyceride level and ameliorate the leptin tolerance, although it has no effect to reduce blood glucose level.

The composition containing the compound shown in the chemical formulae (I) to (II) obtained by using the above-mentioned method, or the physiologically acceptable salt of the compound, the physiologically acceptable hydrate thereof, the physiologically acceptable hydrate thereof as described above may be used to produce the pharmaceutical preparation describe in below. When nobiletin is solely, or a mixture of nobiletin and tangeretin is solely used to prepare the pharmaceutical preparation, crystalline obtained as mentioned above is treated by using the conventional method, and then mixed with excipients and so forth described later. As these pharmaceutical preparations contain these as the active ingredient, there are mentioned such as parenteral preparations such as injectables, suppositories, aerosols, percutaneous, ointment, plaster, spray and so forth, non-parenteral preparations such as tablets, powders, capsules, pills, trochiscus, solutions and other dosage forms.

Here, the above-mentioned tablet includes sugar coated tablets, and buccal tablets; the capsule includes both of hard and soft capsules. The granules contain coated granules. The above-mentioned solution contains suspensions, emulsions, syrups, elixirs, and so forth, and the syrup includes also dry syrups. Note that each preparation includes not only non-sustained preparation, but also sustained preparations. These preparations may be formulated according to the known procedure by using pharmacologically acceptable carrier, excipient, disintegrator, lubricant, colorant, and so forth, for formulating the preparation, described on Japanese Pharmacopoeia. As these carriers or excipients, for example, there are mentioned such as lactose, glucose, sucrose, mannitol, potato starch, corn starch, calcium carbonate, calcium phosphate, calcium sulfate, crystalline cellulose, powdered glycyrrhiza extract, powdered gentian, and so forth.

As a binder, for example, there are mentioned such as the starch, tragacanth gum, gelatin, syrup, polyvinyl alcohol, polyvinylether, polyvinylpyrrolidone, hydroxypropylcellulose, methylcellulose, ethylcellulose, carboxymethylcellulose, and so forth. As the disintegrator, for example, there are mentioned such as starch, agar, powdered gelatin, sodium carboxymethylcellulose, calcium carboxymethylcellulose, crystalline cellulose, calcium carbonate, sodium bicarbonate, sodium alginate and so forth. As the lubricant, for example, there are mentioned such as magnesium stearate, talc, hydrogenated vegetable oil, macrogol and so forth. As the colorant, which is acceptable to be added to the pharmaceutical preparation, can be used with no limitation. Except these additives, a corrigent and so forth cam be used depending on the necessity.

When formulating the tablet or the granule, if necessary, they may be coated by using sucrose, gelatin, hydroxypropylcellulose, purified shellac, gelatin, glycerin, sorbitol, ethylcellulose, hydroxy-propylcellulose, hydroxypropylmethylcellulose, polyvinylpyrrolidone, acetate cellulose phthalate, hydroxypropylmethylcellulose phthalate, methylmethacrylate, methacrylate polymer, and so forth to have single coating or plural coatings. Furthermore, the capsule can be prepared by encapsulating the granule or powdered preparation into the capsule made of ethylcellulose, gelatin, and so forth. When the injectable is prepared by using the above-mentioned compound, the physiologically acceptable salt thereof, or the hydrate thereof, a PH regulator, a buffering agent, a stabilizer, a solubilizing agent, and so forth may be added as needed.
When the preparation for prophylaxis and/or treating the metabolic syndrome as mentioned above is administrated to a patient, the dosage is depending on conditions such as thickness of the symptom, age, weight, and health status and so forth. In general, the preparation is administrated once a day or more at the dosage and administration method as mentioned above. Number of administration and amounts a day can be adjusted depending on the conditions described above optionally.

When the extract is solely used or in combination with any compounds as the active ingredient, these content in the composition is the same as those as mentioned above. When the content of the active ingredients, nobiletin, tangeretin, or the mixture thereof is less than the lower limit, enough effect for ameliorating the metabolic syndrome is not produced. When they are added more than the upper limit, the effect matches to the added amount is not produced. Furthermore, when the amount of them exceeds the upper limit, they show cytotoxicity at the time of dosing, it may cause potential undesirable side effect to a living body being administrated it. Furthermore, the above-mentioned compound, derivatives thereof, physiologically acceptable salt thereof, and physiologically acceptable hydrate thereof are added as necessary, the functional food or healthy food having enhance effect for differentiate the adipose cells may be provided.

The above-mentioned composition, the composition containing the derivative of the compound contained in the composition, physiologically acceptable salt thereof, or physiologically acceptable hydrate thereof is added to, for example, breads, cookies and biscuits, wheat and miscellaneous cereals for being supplemented to rice, noodles such as Japanese wheat noodle, soba noodle, and pasta, dairy product such as cheese, yogurt, jam, mayonnaise, processed soy product such as soybean paste, soy source, tea, coffee and cocoa, nonalcoholic beverage such as soft drinks and fruits juice, alcoholic beverage such as medicated liquor, snacks such as candy, and chocolate, chewing gum, Japanese cracker, sweets made from azuki-bean such as azuki-bean jelly, to produce the functional food. They may be added to animal feed to prepare a functional animal feed. When the composition is added to the yogurt, soy source, beverages and so forth, an auxiliary agent for solubilizing or the stabilizer may be added optionally to avoid the precipitation of crystalline of the present composition in them.

Also, the composition of the present invention may be used solely or in combination of two or more, and to formulate the powdered agent, the granule, the tablet or the capsule, to prepare the health food. Here, in order to form the powdered composition of the present invention, the extract obtained in the production process may be condensed, and dried by using the method such as lyophilization, spray-drying, vacuum-drying and so forth; and then dried extract is pulverized into fine powder by using a sample mill, blender, mixer or the like. Corn starch, dextrin, cyclodextrin, oyster shell powder may be added to the powder as needed.

Alternatively, the binder is optionally added to the powder obtained as described above and compressed to formulate the tablet. After formulation of the tablet, it may be coated by using the coating agent such as sucrose, gelatin and so forth to formulate the sugar-coated tablet, or coated by other coating agent to formulate enteric coated tablet. Furthermore, the powder obtained as describe above may be granulated by using the conventional method to formulate the granule. The powder or granule as mentioned above is encapsulated into capsules in a proper amount to formulate the capsule. In the specification, note that the terms, "functional food" or "healthy food", do not include fruits itself of the plant such as citrus, which spontaneously contains the composition as mentioned above. However, the food produced by adding the above-mentioned composition to the fruits is not excluded the scope of the present invention.

### Example 1

### 1. Preparation of test substance and so forth

### (1-1) Extraction method of the extract from peel of citrus fruit belonged to Mikan-ku citrus

Fruits of Shiikuwasha (*Citrus depressa*) were purchased from Oogimi of Okinawa prefecture. Peel of Shiikuwasha fruits were dried for three days under the sunshine to obtain dried Shiikuwasha peel. Citrus unshiu fruit was purchased from those produced in Ehime prefecture. The peel of citrus unshiu was dried similarly for three days under the sunshine to obtain dried citrus unshiu peel.
Eight kg of the respective dried peels was immersed in 40 L of MeOH to perform extraction for 14 days at 4 °C. Crude extracts were obtained by filtration, and then whole extracts were condensed by using an evaporator to obtain a condensed solution. The condensed solution was subjected to liquid-liquid partition by using 4L of water/ethyl acetate (1/1). Ethyl acetate phase were then subjected to the liquid-liquid partition by using 4L of 90 % MeOH-water/Hexane (1/1).

Obtained 90 % MeOH phase was subjected to ODS silica gel open column (Cosmosil 75C₁₈-OPN) to be eluted by using the step-gradient method wherein MeOH contents in water-MeOH mobile phase was increased 20 % by 20 %. By this elution, 3 fractions, 60 % MeOH/80 % MeOH/ 100 % MeOH were obtained. The 80 % MeOH fraction was used as the extract from the extract from citrus peel of Mikan-ku citrus.

### (1-2) Composition of the extract of citrus peel in citrus class

The extract was subjected to HPLC analysis by using known method. HPLC conditions were as follows:

| | |
|---|---|
| HPLC system | : UV-8011 HPLC (TOSOH Corp.) |
| Detector | : UV Detector |
| Column | : Cholester Waters i.d. 4.6mm × 250 mm (Waters Corp.) |
| Elution buffer | : 75% MeOH/water |
| Solute concentration | : 0.1 mg/mL |
| Injection volume | : 0.005 mL |
| Flow rate | : 1.0 mL/min. |
| Detection wave length | : UV 215 nm |
| Range | : 1000 mV |

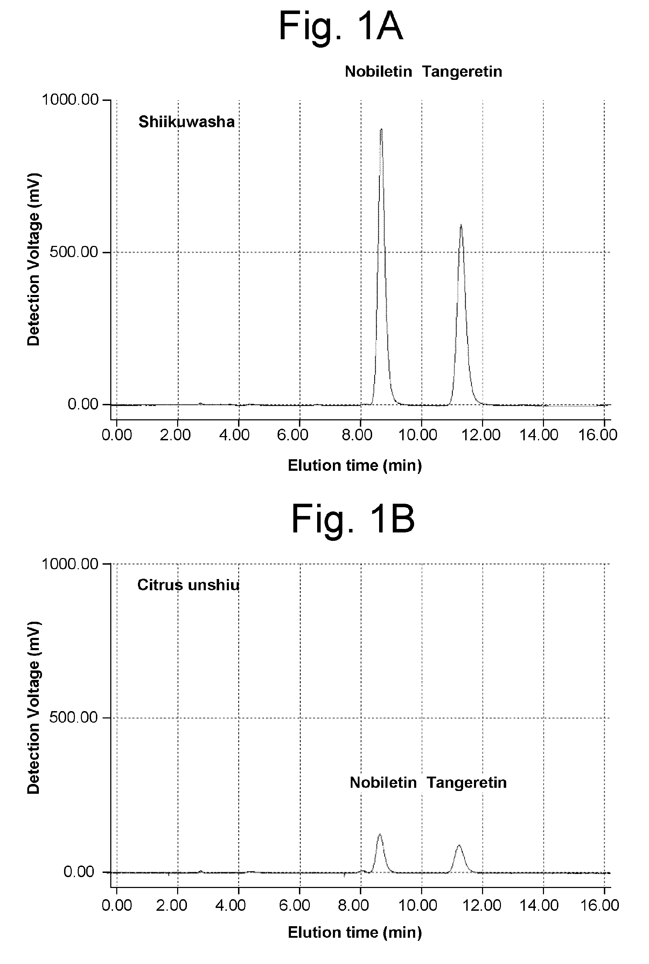

As shown in Fig. 1, nobiletin and tangeretin were detected. As clearly shown in a comparison of Fig. 1A and Fig. 1B, nobiletin and tangeretin in Shiikuwasha were extracted in high concentration than that in citrus unshiu. In the extract from Shiikuwasha, content ration of nobiletin and tangeretin was 50 % /50 %.

### Hereinbelow, the extract which was obtained from Shiikuwasha peel by using the above-mentioned method and containing 50 % of nobiletin (Nobiletin) and 50 % of tangeretin (Tangeretin) was used as the Shiikuwasha peel extract (Shiikuwasha Peel Extract; SPE).

### Example 2

### 2. Feedstuff for test animals and a method for gene expression level (2-1) The feedstuff for test animals

As Normal feedstuff for mice (normal feedings), CRF-1 (Oriental Yeast Co., Itd) was purchased. As preparation additives for the low fat diet and the high fat diet, casein, tallow, β-cornstarch, α-cornstarch, alimentary fiber, minerals and vitamins (all of them were provided by Oriental Yeast Co., Itd) were purchased, and used by adding in the amount (w/w %) shown in below. Furthermore, sucrose, L-cystine, choline bitartrate, and t-butylhydroquinine (all of them were provided by Wako Pure Chemical Industries, Ltd) were purchased, and added to CRF-1 for feeding in the amount shown in the following table.
Table 1 shows the composition of feedings, the low fat diet, the high fat diet and SPE added diet in the example 3. Each value shows weight % (w/w %). The component with *1 was purchased from Wako Pure Chemical Industries, Ltd. Chemicals other than sucrose, L- cystine, choline bitartrate, and t- butylhydroquinine were purchased from Oriental Yeast Co., Itd.

**[Table 1]**

| Components | low fat diet | high fat diet | high fat diet +SPE 1% | high fat diet +SPE 1.5% |
|---|---|---|---|---|
| Casein | 14 | 14 | 14 | 14 |
| tallow | 4 | 40 | 40 | 40 |
| β-cornstarch | 46.57 | 10.57 | 10.57 | 10.57 |
| α-cornstarch | 15.5 | 15.5 | 15.5 | 15.5 |
| sucrose *1 | 10 | 10 | 9 | 8.5 |
| Cellulose powder | 5 | 5 | 5 | 5 |
| Mineral mixture | 3.5 | 3.5 | 3.5 | 3.5 |
| Vitamin mixture | 1 | 1 | 1 | 1 |
| L-cystine *1 | 0.18 | 0.18 | 0.18 | 0.18 |
| choline bitartrate *1 | 0.28 | 0.28 | 0.28 | 0.28 |
| t-butylhydroquinine *1 | 0.0008 | 0.0008 | 0.0008 | 0.0008 |
| Shiikuwasha (SPE) | 0 | 0 | 1 | 1.5 |
| Total | 100 | 100 | 100 | 100 |

### (2-2) Determination method of gene expression level

mRNA expression level was determined by using real time PCR as follows.
According to the direction attached with ISOGEN (Nippon Gene Co., Ltd), RNA was extracted from white adipose tissue, liver, and skeletal muscle. Next, cDNA was prepared from 1 µg of RNA by using Reverse Transcription System kit (Promega, USA). Subsequently, according to the direction attached with FastStart universal SYBR (Registered trademark) Greenmaster PCR kit reagent (Roche Diagnostics, Germany), primers suitable for each intended gene in the following experiments were synthesized. Real time PCR system 7700 HT (Applied Biosytems, USA) was used for the measurement, the cDNA was used as templates for the real time PCR. Reaction conditions were followed to those for the PCR kit reagents. Quantitative analysis of RNA level was performed by Comparison Ct method, using glyceraldehyde 3-phosphate dehydrogenase (GAPDH) as the standard.

### Example 3

### 3. Measurement of SPE effect against obesity in high fat diet ingestion

The animals used in the following examples were fed either of the low fat diet (low fat diet; fat content 4%) or the high fat diet (high fat diet; fat content 40 %). As the high fat diet, three kinds of diet, without SPE, 1% SPE added, 1.5 % SPE added were prepared. In the example 3, the group to which the low fat diet was administrated is shown as LFD administration group, the group to which the high fat diet without SPE was administrated is shown as HFD administration group, the group to which the high fat diet with 1 % SPE was administrated is shown as HFD + 1SPE administration group, and the group to which the high fat diet with 1.5 % SPE is administrated was shown as HFD + 1.5SPE administrated group. In the following tables, they are respectively shown as LFD, HFD, HFD + 1SPE, and HFD + 1.5SPE.
As animals for the experiments, 7 week age of C57BL/6 mice were purchased from Charles River Laboratories Japan Inc. They were preliminary kept with the normal feed for a week, and then, they are classified into 4 groups (n= 9, 3 mice/cage) for 5 week experiments. They were maintained at the constant temperature (23 ± 5 °C), a cycle of 12 hour light/12 hour dark ad libitum feeding.

Nobiletin reference standard and tangeretin reference standard, haematoxylin, eosine, and carboxymethylcellulose were purchased from Wako Pure Chemical Industries, Ltd. Glucose for the oral glucose tolerance test was purchased from Sigma Aldrich Co. LLC. Pioglitazone was purchased from Takeda Pharmaceutical Company Limited.

### (3-1) Change of SPE ingestion, weight and ingestion amounts when the high fat diet was administrated

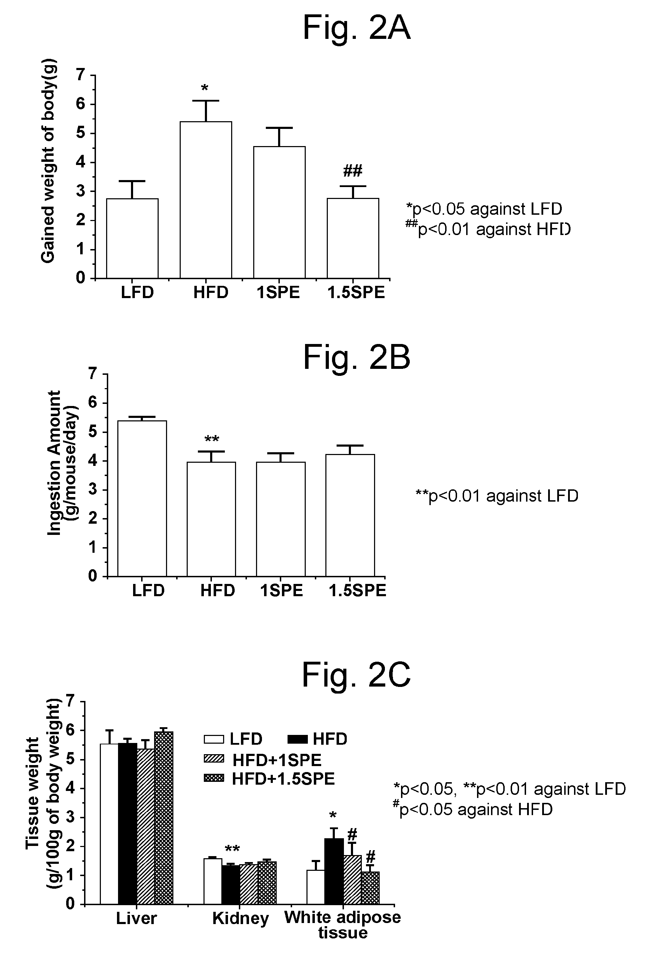

Body weights of the mice were measured once a week and ingestion amounts of each group were measured twice a week. The ingestion amounts in 24 hours/3 mice /cage were measured and obtained as mean average of each group for 5 weeks. The SPE ingestion and body weight change of the mice in each group and that of ingestion amounts were shown in the table 2, Figs. 2A and 2B. In the following tables, significant difference against the LFD administration group is shown as ^{*}, that against the HFD group is shown as ^{#} respectively.

**[Table 2]**

| | Weight gain and ingestion amount | | | |
|---|---|---|---|---|
| | LFD | HFD | HFD + 1SPE | HFD + 1.5SPE |
| Weight gain (g) | 2.42 ± 0.54 | 5.03 ± 0.65^{***} | 4.39 ± 0.65 | 2.55 ± 0.40^{#} |
| Ingestion amount (g/day) | 5.40 ± 0.13 | 3.96 ± 0.37^{**} | 3.96 ± 0.31 | 4.24 ± 0.30 |

| | | | | |
|---|---|---|---|---|
| ^{**}: p <0.01 ^{***}: p <0.005 ^{#} : p <0.05 | | | | |

After 5 weeks, HFD administration group showed the significant weight gain compared to that of the LFD administration group (p <0.05). However, HFD + 1SPE did not show any significant difference compared to the HFD administration group. HFD + 1.5SPE administration group showed significant low value compared to HFD administration group (p < 0.01).
The ingestion amount of HFD administration group was significantly low compared to that of LFD administration group (p <0.01). On the other hand, compared to HFD administration group, in both of HFD + 1SPE administration group and HFD + 1.5SPE administration group, there was no significant difference of the ingestion amount change.
Accordingly, the ingestion amount of the high fat diet group did not decreased by adding 1.0 to 1.5 % of SPE to feedings, but the body weight gain of the mice was reduced by adding 1.5 % SPE. Since there was no change of the ingestion amount, it was thought that the reduction of the weight gain was not caused by reduction of calorie intake (Fig. 2B).

### (3-2) Effect to each tissue weight by SPE ingestion when the high fat diet was ingested

After the experiment was finished, all of the mice in the group were dissected. The tissue and organ weight were shown in the following table 3 and Fig. 2C (mean ± standard deviation, n= 9).

**[Table 3]**

| Tissue | Tissue weight (g/body weight 100g) | | | |
|---|---|---|---|---|
| | LFD | HFD | HFD + 1SPE | HFD + 1.5SPE |
| Liver | 5.54 ± 0.46 | 5.57 ± 0.15 | 5.36 ± 0.31 | 5.96 ± 0.13 |
| Kidney | 1.58 ± 0.06 | 1.34 ± 0.06^{**} | 1.37 ± 0.06 | 1.48 ± 0.07 |
| White adipose tissue | 1.18 ± 0.32 | 2.28 ± 0.35^{*} | 1.69 ± 0.44 | 1.12 ± 0.23^{#} |

| | | | | |
|---|---|---|---|---|
| ^{*}: p <0.05, ^{**}: p <0.01 ^{#} : p <0.05 | | | | |

After 5 weeks, there was no significant difference between the liver weight of HFD administration group and that of LFD administration group. Also, there was no significant difference between the HFD administration group and the HFD + 1PE administration group, or the HFD + 1.5SPE administration group. Accordingly, it was demonstrated that the addition of 1.0 to 1.5 % of SPE did not affect the liver weight.
In HFD administration group, kidney weight was significantly low compared to that of LFD administration group (p <0.01). On the other hand, in HFD + 1 SPE administration group and HFD + 1.5SPE administration group, there was no significant increase or decrease of the kidney weight of these groups compared to that of HFD ingestion group.
Accordingly, it was demonstrated that the decrease of the kidney weight was caused by HFD ingestion, not but addition of 1.0 to 1.5 % SPE.
Alternatively, the weight of the white adipose tissue excised around from genitals and kidneys of the HFD administration group were significantly high compared to those of LFD administration n group (p <0.05). There were no significant differences between the weight of those of HFD + 1SPE administration group and HFD administration group. However, those of HFD + 1.5SPE administration group were significantly low compared to those of HFD administration group (p <0.001). Accordingly, it was demonstrated that SPE inhibits the white adipose tissue weight gain when the high fat diet was given to the mice.

### (3-3) Blood neutral fat level change and so forth by ingestion of SPE

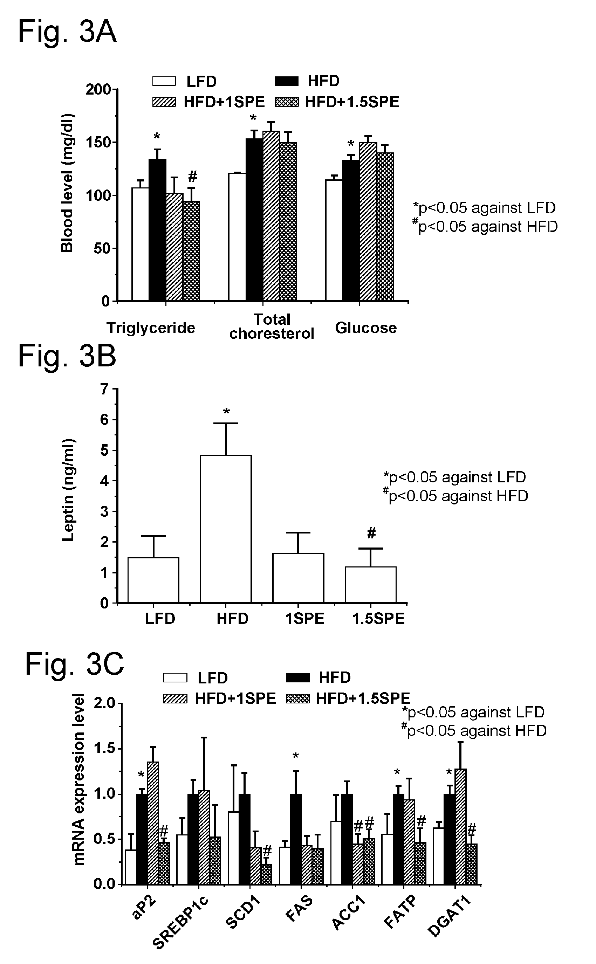

After completion of the experiment, items shown in the table 4 were measured. At the completion date of the experiment (5 weeks after the beginning of the administration), all of the mice were starved for 16 hours; then 1 mL of blood was collected from their tail veins. Obtained blood was centrifuged by 3,000 x g at 4 °C for 15 minutes to separate plasma. As lipids in plasma, concentrations of neutral fat (TG) and total cholesterol (T-CHO) were measured (n= 9).
TG was measured by using Triglyceride E-test Wako; T-CHO was measured by using cholesterol E-test Wako; blood sugar (glucose) was measured by using Glucose CII test Wako (all of them are purchased from Wako Pure Chemical Industries, Ltd.). Results were shown in the table 4, Figs 3A and 3B.

**[Table 4]**

| | Blood Concentration of neutral fat and so forth | | | |
|---|---|---|---|---|
| Items measured | LFD | HFD | HFD + 1SPE | HFD + 1.5SPE |
| Glucose(mg/dl) | 114.42 ± 4.41 | 133.05 ± 4.87^{*} | 149.67 ± 6.25 | 140.15 ± 7.58 |
| TG(mg/dl) | 106.85 ± 7.27 | 134.47 ± 8.76^{*} | 101.75 ± 15.13 | 94.24 ± 12.88^{#} |
| T-CHO(mg/dl) | 120.43 ± 0.93 | 153.46 ± 7.87^{*} | 160.33 ± 8.97 | 149.90 ± 9.85 |
| Leptin(ng/mL) | 1.49 ± 0.71 | 4.82 ± 1.05^{*} | 1.86 ± 0.68 | 1.18 ± 0.60^{#} |
| adipo(µg/mL) | 4.15 ± 0.71 | 3.58 ± 0.26 | 3.28 ± 0.33 | 3.00 ± 0.22 |

| | | | | |
|---|---|---|---|---|
| adipo: Adiponectin ^{*}: p <0.05 ^{#}:p <0.05 | | | | |

Blood TG level, T-CHO level, and glucose level of HFD administration group were significantly high compared to those of LFD administration group (p <0.05). There was no significant difference of TG concentration between HFD administration group and that of HFD + 1SPE administration group. However, that of HFD + 1.5SPE administration group was significantly low compared to that of HFD administration group (p <0.05). On the other hand, there were no significant differences of the concentrations of T-CHO and glucose between HFD administration group and HFD + 1SPE administration group or between HFD administration group and HFD + 1.5SPE administration group.
As mentioned above, it was demonstrated that the addition of 1.0 to 1.5 % SPE inhibited the increase of blood triglyceride concentration of the high fat diet administration group, but did not affect to the total cholesterol concentration and glucose concentration in blood.

Blood Adiponectin level was measured by using Quantikine Mouse Adiponectin/Acrp30 (R&D Systems, USA) based on enzyme immunoassay. There were no significant differences of the Adiponectin concentration in blood between HFD administration group and the LFD administration group, also between the HFD administration group and the HFD + 1SPE administration group, or HFD administration group and the HFD + 1.5SPE administration group.
As mentioned above, it was demonstrated that the addition of 1.0 to 1.5 % SPE inhibited the increase in plasma Adiponectin concentration of the high fat diet administration group. As shown in the example 5, in the diabetes model mice, plasma Adiponectin concentration was increased about 2 or 3 times higher compared to the LFD administration group, healthy mice, by nobiletin administration.

Blood leptin level was measured by using Quantikine Mouse leptin (R&D Systems, USA) based on the enzyme immunoassay. HFD administration group showed significantly high level of blood leptin level compared to LFD administration group (p <0.05). The blood leptin level of HFD + 1SPE administration group was almost half of that of HFD administration group. However, there was no significant difference between them. In contrast, HFD + 1.5SPE administration group showed the significantly low level (p < 0.05).
As mentioned above, it was demonstrated that the addition of 1.0 to 1.5 % SPE inhibits the leptin resistance by the high fat diet ingestion. This means that appetite is normally regulated under the low level of leptin.

### (3-4) Effects of SPE ingestion to adipocytes in the white adipose tissue

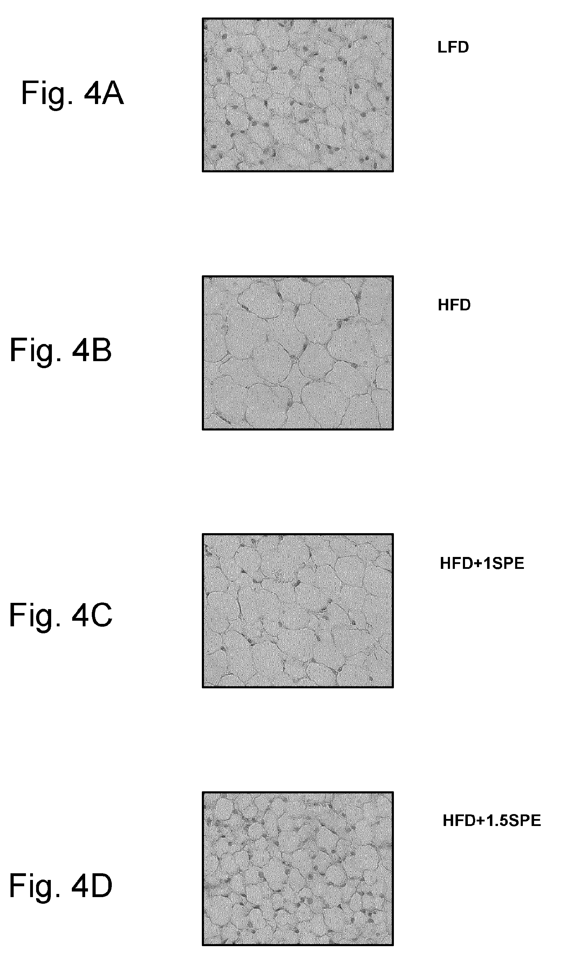

After termination of the experiment, adipose tissue of epididymis was isolated from each mouse in the group to prepare sections to be stained with hematoxylin and eosin. These sections were observed by using a microscope of 200 times power, and cell numbers per unit area to compare (n= 9). Results were shown in the table 5 and Fig. 4.

**[Table 5]**

| Groups | Cell number/unit area) |
|---|---|
| LFD | 56.50±0.50 |
| HFD | 31.67±3.84^{*} |
| HFD + 1SPE | 36.00±2.68 |
| HFD + 1.5SPE | 50.60±3.37^{#} |

| | |
|---|---|
| ^{*}: p <0.05 ^{#}: p <0.05 | |

In the HFD administration group, adipocyte size was significantly large compared to the LFD administration group (p <0.05) (Figs. 4A and 4B). In contrast, there was no significant difference of the adipocyte size between the HFD administration group and the HFD + 1SPE administration group (Fig. 4C). However, the HFD + 1.5SPE administration group showed the significantly small size of the adipocytes (p < 0.05) (Fig. 4D). Because of this, it was demonstrated that SPE has an effect to inhibit the enlargement of the adipocyte by the high fat diet ingestion. Alternatively, this result indicates that SPE has the effects to reduce the size of enlarged white adipose tissue and adipocytes in the white adipose tissue. Such an effect is not observed in the diabetes model animal used in the example 5 mentioned below. Namely, this showed that when 200 mg/mL of nobiletin was administrated, the enlarged adipocytes of the white adipose tissue in development of diabetes were not reduced in size; however, in the status of obesity caused by the ingestion of the high fat diet, namely, in precritical of diabetes, they were reduced in size by ingesting 1.5 % SPE against the total amount of the feedings.
It is considered from these results that SPE promotes the differentiation induction of the adipocytes to divide the enlarged adipocytes, or promotes to downsize the adipocytes in the white adipose tissue by replacing the enlarged adipocytes with newly differentiated adipocytes to ameliorate insulin resistance in precritical diabetes.

### (3-5) Effects of SPE ingestion to the gene expression level in the white adipose tissue

mRNA expression levels of genes related to lipid biosynthesis of the mice in each group, aP2, SREBP1c, SCD1, FAS, ACC1, FATP, and DGAT1 were determined (n= 9). Results were shown in the table 6 and Fig. 3C.

**[Table 6]**

| Genes related to lipid biosynthesis | Expression Amount (Ratio) | | | |
|---|---|---|---|---|
| | LFD | HFD | HFD + 1SPE | HFD + 1.5SPE |
| aP2 | 0.38±0.18 | 1.00±0.05^{*} | 1.36±0.17 | 0.46±0.05^{#} |
| SREBP1c | 0.55±0.19 | 1.00±0.16 | 1.04±0.58 | 0.52±0.36 |
| SCD1 | 0.80±0.51 | 1.00±0.23 | 0.41±0.18 | 0.22±0.08^{#} |
| FAS | 0.42±0.07 | 1.00±0.26^{*} | 0.43±0.11 | 0.40±0.15 |
| ACC-1 | 0.70±0.30 | 1.00±0.14 | 0.44±0.12^{#} | 0.51±0.10^{#} |
| FATP | 0.55±0.23 | 1.00±0.09^{*} | 0.94±0.24 | 0.47±0.16^{#} |
| DGAT1 | 0.62±0.07 | 1.00±0.10^{*} | 1.28±0.30 | 0.45±0.10^{#} |

| | | | | |
|---|---|---|---|---|
| ^{*}: p <0.05 ^{#} : p < 0.05 | | | | |

In the HFD administration group, mRNA expression levels of aP2, FAS, FATP, DGAT1 after termination of the experiment were significantly enhanced compared to the LFD administration group (all of them p < 0.05). mRNA expression levels of SREBP1c, SCD1, and ACC1 did not show significant differences. However, their levels showed upward trend.
No significant differences of the mRNA expression levels of aP2, SCD1, ACC1, FATP and DGAT1 were observed between the HFD administration group and HFD + 1SPE administration group. However, significant reductions were observed between the HFD administration group and HFD + 1.5SPE administration group (p < 0.05). In both of the HFD + 1SPE administration group and the HFD + 1.5SPE administration group, mRNA level of ACC1 was significantly reduced (p < 0.05). Note that in both of the HFD + 1SPE administration group and HFD + 1.5SPE administration group, mRNA levels of SREBP1c and FAS were downward trend, but no significant differences.
As mentioned above, it was indicated that SPE has the effect to inhibit the expressions of the lipid biosynthesis related genes which were enhanced by the ingestion of the high fat diet.

### Example 4

### (1) Reagents

All of nobiletin reference standard, tangeretin reference standard, hematoxylin, eosin and carboxymethylcellulose were purchased from Wako Pure Chemical Industries, Ltd. Glucose for oral glucose tolerance test was purchased from Sigma-Aldrich Corporation, and Pioglitazone was purchased from Takeda Pharmaceutical Co., Ltd.

### (2) Purification of nobiletin and tangeretin from SPE

A part of 80 % MeOH fraction obtained in the example 2 was condensed, and its solute concentration was adjusted so as to become 900 mg/mL by using 100 % MeOH. Subsequently, it was subjected to the preparative chromatography under the following conditions to obtain seven fractions.

Preparative column: 5C₁₈-AR-II column (NACALAI TESQUE, INC.)
Elution sorbent: 70 % MeOH
Solute concentration: 900 mg/mL
Injection volume: 3mL
Fraction volume: 10mL
Flow rate: 5nL/min.
Detection wavelength: UV 215 nm
Range: 20mV
As the internal standard, reference standards of nobiletin and tangeretin were used (concentration= 100 µg/mL). Compared to retention time of the peak in each fraction (RT: retention time (min)) and those of the reference standards, nobiletin and tangeretin were identified to obtain the contents in the fractions. After that, each purified fraction was dried by using a large size of a rotary evaporator, about 165 mg (55%) of nobiletin and about 135 mg (45%) of tangeretin were respectively obtained.

### Example 5

### 5. Effect of nobiletin for Type II diabetes model mice (ob/ob)

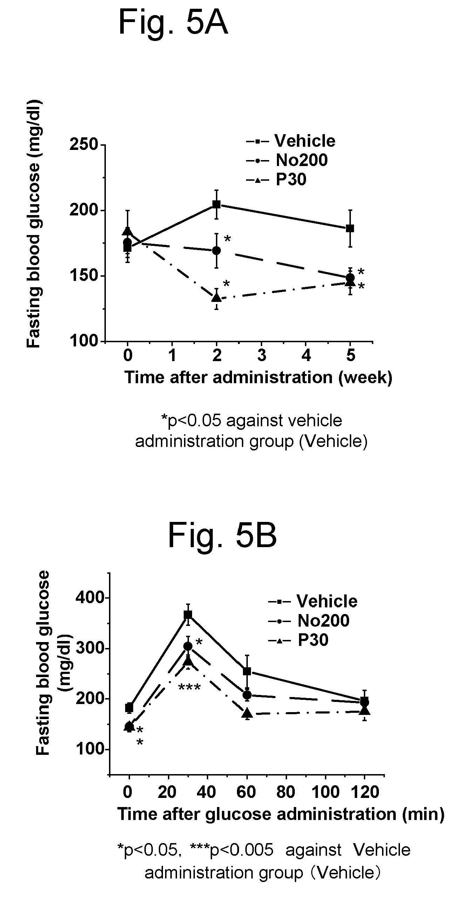

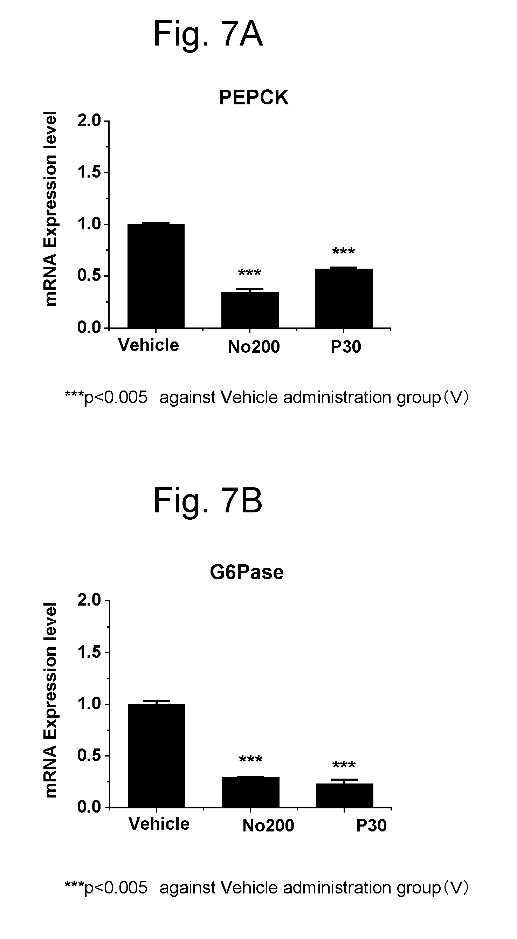

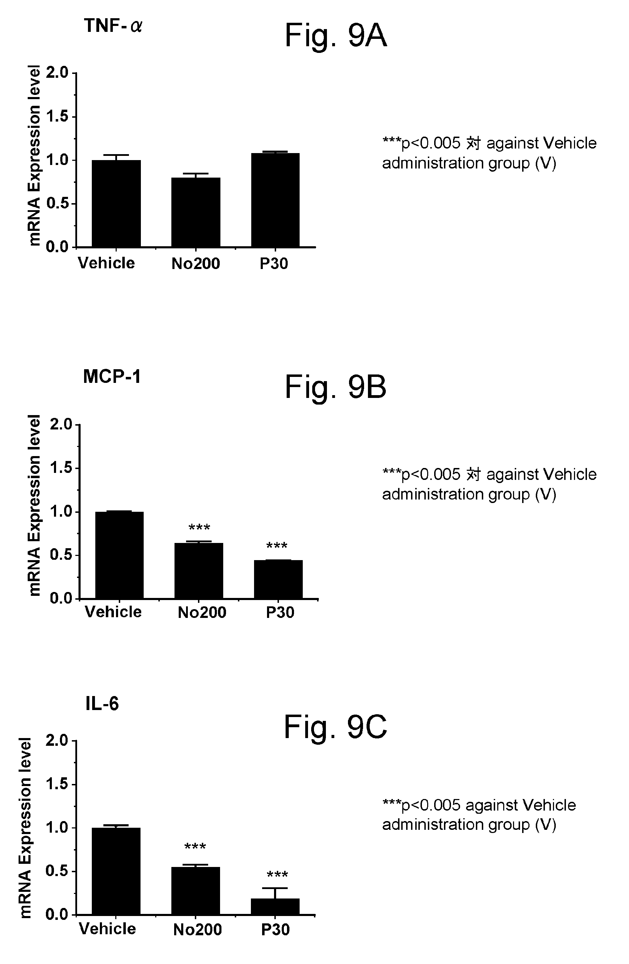

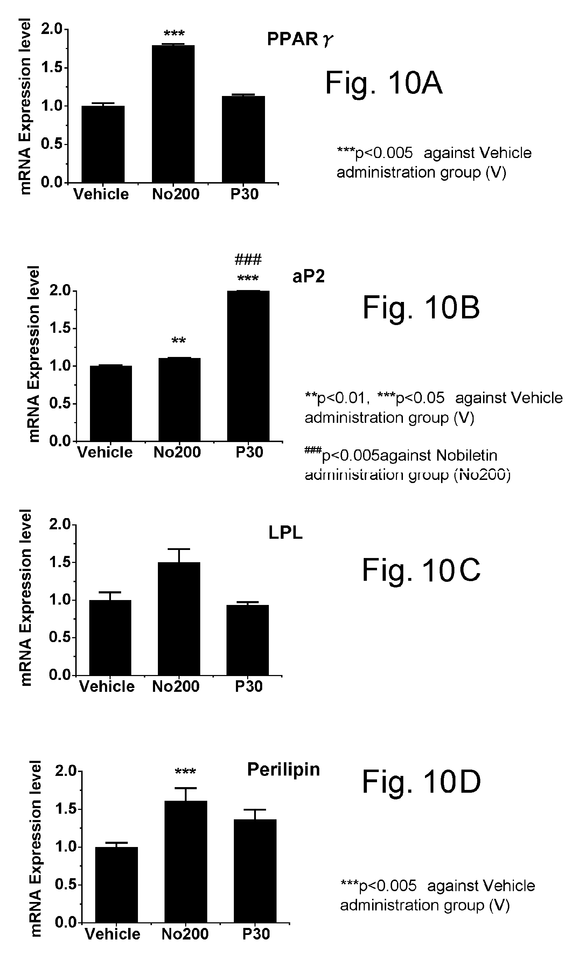

Diabetes model mice and the experimental system were as follows. As the diabetes model mice, 7 week age of male C57BL/6J-ob/ob(hereinafter, it was referred to as "ob/ob") were purchased from Charles River Laboratories Japan Inc., and used as 10 mice /group. Conditions of the preliminary breeding and breeding in the experiment were the same as those of the example 3, and CRF-1 was used as the feeding.
For the negative control group (from Figs. 5 to 10, it was shown as Vehicle; hereinafter, it is referred to as "solvent administration group"), 0.3 % of CMC aqueous solution was administrated orally for 5 weeks in everyday at the dosage of 0.01mL/g body weight. For the positive control group (in Figs. 5 to 10 and hereinafter, it was referred to as "P30 administrated group"), 3 mg of Pioglitazone was suspended in 0.3% CMC aqueous solution so as to be orally administrated at the dosage of 0.01mL/g body weight for 5 weeks in everyday ( dosage: 30mg/kg body weight/day).
For the sample ingestion group (in Figs. 5 to 10 and hereinafter, it is referred to as "No200 ingestion group"), 20 mg of nobiletin purified as mentioned above (1) was suspended in 0.3% of carboxymethylcellulose (CMC) aqueous solution so as to be orally administrated at the dose of 0.01mL/g body weight for 5 weeks in everyday (the dose: 200mg/kg/day).

### (5-1) Effect of nobiletin ingestion to the body weight and others of diabetes model mice

### (5-1-1) Effect of nobiletin ingestion to the body weight and the like

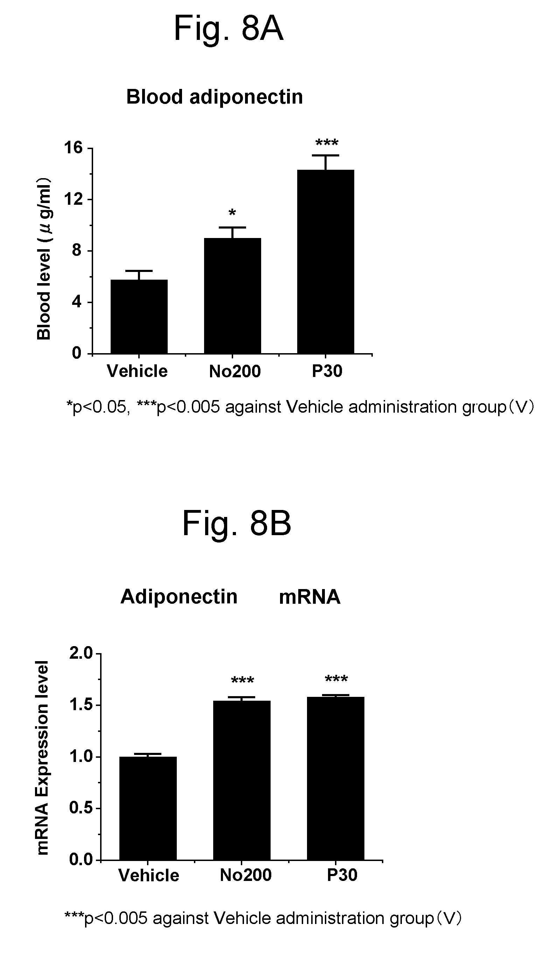

The body weight of mice, ingestion amount, weight of the white adipose tissue and liver weight of each mice were measured as the same as described in the example 3. The levels of TG, T-CHO, and Adiponectin in blood were also as the same as those described in the example 3. Results were shown in the table 7 (n= 10). The results of the Adiponectin levels were particularly shown in Fig. 8A.
The blood insulin level was measured as described in below. On the date of termination of the experiment, 1 mL of the blood was collected from the tail vein of each mouse. Obtained blood was centrifuged by 3,000 x g at 4 °C for 15 minutes to separate plasma. As lipids in plasma, concentrations of neutral fat (TG) and total cholesterol (T-CHO) were measured. Plasma insulin level was measured by using rebis insulin mouse (type U) (Shibayagi Co.Ltd.) based on the enzyme-immunoassay.

**[Table 7]**

| Items measured | Measurement Results | | |
|---|---|---|---|
| | Vehicle | No200 | P30 |
| Gained body weight (g) | 9.37±0.74 | 9.33±0.49 | 10.83±0.58 |
| Ingestion amount (g/day) | 5.36±0.16 | 5.00±0.11 | 4.96±0.13 |
| white adipose tissue (g) | 5.94±0.12 | 5.69±0.17 | 5.70±0.17 |
| Liver weight (g) | 3.17±0.13 | 3.07±0.17 | 3.15±0.13 |
| TG in blood (mg/dl) | 83.88±11.32 | 69.15±2.17 | 70.80±2.95 |
| T-CHO in blood (mg/dl) | 166.20±6.26 | 164.13±6.93 | 141.65±6.99^{*} |
| Insulin in blood (ng/mL) | 9.05±1.20 | 7.16±0.97 | 6.99±0.71 |
| Adiponectin in blood (µg/mL) | 5.75±0.71 | 8.98±0.86^{*} | 14.31±1.13^{***} |

| | | | |
|---|---|---|---|
| ^{*}: p <0.05 ^{***}: p <0.005 | | | |

In either group, there were no significant differences between the body weight gain, the ingestion amount, the white adipose tissue weight, and liver weight. These mean that the above-mentioned amounts of nobiletin ingestion has no effect to the body weight gain, ingestion amount, the white adipose tissue weight, and the liver weight of the diabetes model mice.
TG levels in blood both in No200 administration group and P30 administration group were lower than that of the vehicle administration group; however, there were no significant differences. By this, it was indicated that the TG levels in blood of diabetes model mice were reduced by ingesting 200 mg/kg of nobiletin in a certain period similarly to the case when Pioglitazone was ingested.

T-CHO level in blood of P30 administration group was significantly reduced compared to that of sorbent administration group (p <0.05). However, between the T-CHO level of the solvent administration group and that of the No200 administration group, there was no significant difference. By this, it was demonstrated that the nobiletin ingestion of the above-mentioned concentration has no effect to the level of T-CHO in blood.
Blood insulin levels of No200 administration group and P30 administration group were downward trends compared to that of the sorbent group. However, there were no significant differences. On the other hand, the Adiponectin level in blood of the P 30 administration group was equal or more than 2.5 times higher than that of the solvent administration group. However, the level of Adiponectin of No200 administration group was not so high.
By this, it was demonstrated that the blood insulin level has trend to reduce similarly to that of Pioglitazone administration when the above-mentioned concentration of nobiletin was administrated in a certain term; however, it did not increase the blood Adiponectin level compared to that when Pioglitazone administration.

### (5-1-2) Effects to the fasting blood glucose level by nobiletin ingestion

Two weeks or 5 weeks from the beginning of the administration, the fasting blood glucose was measured (n=10). The measurement of the blood glucose level was carried out as the same as that of the example 1. The results were shown in the table 8 and Fig. 5.

**[Table 8]**

| Time after administration (week) | Blood glucose level (mg/dl) | | |
|---|---|---|---|
| | Vehicle | No200 | P30 |
| 0 | 171.21±10.78 | 175.61±11.44 | 183.61±16.51 |
| 2 | 204.51±10.78 | 169.22±13.22^{*} | 132.50±7.86^{*} |
| 5 | 186.24±14.06 | 148.46±7.56^{*} | 144.67±8.96^{*} |

| | | | |
|---|---|---|---|
| ^{*}: p <0.05 | | | |

Both at 2 weeks and 5 weeks of the administration, the level of the No200 administration group was significantly lower compared to that of the vehicle administration group (p <0.05). By this, it was demonstrated that nobiletin has the effect to reduce the fasting blood glucose level of the diabetes model mice.

### (5-1-3) Effect by nobiletin ingestion to glucose tolerance

The oral glucose tolerance test (OGTT) for the diabetes model mice (ob/ob) to which the nobiletin and others were administrated was subjected to the glucose tolerance test for studying the effects.
From 4 weeks after the start of the administration, the mice in each group were fasted for 16 hours, and then they were administrated 2 g/kg body weight of glucose per os. After the administration of glucose, 1 mL of blood was collected from the tail vein at the time point of 0, 30, 60 and 120 minutes. Collected blood was centrifuged at 4 °C, 3,000 x g, for 15 minute to obtain plasma (n=10). The blood glucose level was measured as the same as that described in the example 1. The results were shown in the table 9 and Fig. 5B.

**[Table 91**

| Time after administration (min.) | Blood glucose level (mg/dl) | | |
|---|---|---|---|
| | Vehicle | No200 | P30 |
| 0 | 182.14±9.56 | 145.26±7.56 | 144.67±8.96 |
| 30 | 367.29±20.78 | 304.41±20.23^{*} | 273.55±14.15^{**} |
| 60 | 254.41±32.40 | 207.47±11.56 | 170.60±4.45 |
| 120 | 196.22±20.85 | 192.57±9.01 | 175.79±18.06 |

| | | | |
|---|---|---|---|
| ^{*}: p <0.05 ^{**}: p <0.01 | | | |

Immediately after the administration of glucose, the blood glucose level were significantly low in both of the No200 administration group and P30 administration group compared to vehicle administration group (p < 0.05). Half an hour later of the administration, their levels were similar to those (p <0.05 and p <0.005). This showed that nobiletin has an effect to ameliorate glucose tolerance of the diabetes model mice.

### (5-2) Nobiletin ingestion and the expression levels of genes in the white adipose tissue and the liver

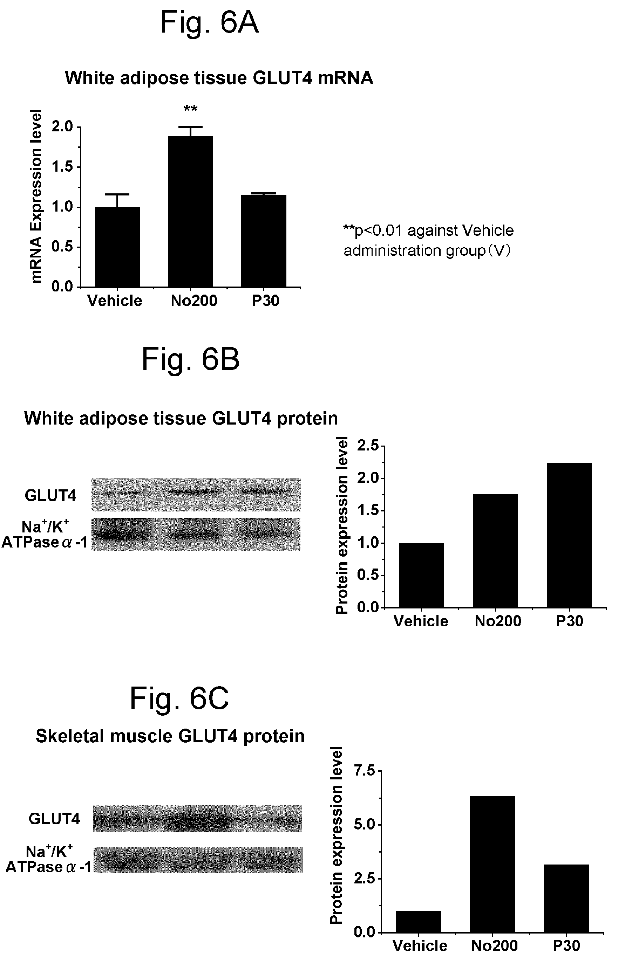

The effects by nobiletin ingestion to the mRNA expression in the white adipose tissue and the liver were measured (n=10). The results were shown in the table 10, Figs. 6A, 7A, 7B, 8B, 9A to 9C, and 10A to 10D.

**[Table 10]**

| | Gene expression level (ratio) | | |
|---|---|---|---|
| [Tissue] Genes | Vehicle | No200 | P30 |
| [W] GLUT4 | 1.00±0.16 | 1.88±0.12^{**} | 1.15±0.02 |
| [W] Adiponectin | 1.00±0.03 | 1.54±0.04^{***} | 1.58±0.02^{***} |
| [W] TNF-α | 1.00±0.06 | 0.80±0.048 | 1.08±0.01 |
| [W] MCP-1 | 1.00±0.04 | 0.64±0.02^{***} | 0.44±0.10^{***} |
| [W] IL-6 | 1.00±0.02 | 0.55±0.03^{***} | 0.19±0.12^{***} |
| [W] PPARγ | 1.00±0.04 | 1.79±0.02^{***} | 1.13±0.02 |
| [W] aP2 | 1.00±0.01 | 1.10±0.01^{**} | 2.03±0.01^{***} |
| [W] LPL | 1.00±0.10 | 1.50±0.18 | 0.93±0.04 |
| [W] Perilipin | 1.00±0.06 | 1.61±0.17^{***} | 1.37±0.13 |
| [L] PEPCK | 1.00±0.01 | 0.34±0.03^{***} | 0.57±0.01^{***} |
| [L] G6Pase | 1.00±0.02 | 0.29±0.01^{***} | 0.23±0.04^{***} |

| | | | |
|---|---|---|---|
| In the table, [W] shows the white adipose tissue, and [L] shows the liver. ^{*}: p <0.05, ^{**}: p <0.01, ^{***}: p <0.005 | | | |

After the termination of the administration period for 5 weeks, mRNA expression levels of respective genes shown in the table 10 were determined by using real time RT-PCR method. The real time RT-PCR was carried out according to an operation manual provided by ISOGEN (NIPPON GENE CO., LTD); RNA was extracted from the white adipose tissue and the liver. Next, cDNA was prepared from using 1 µg of RNA by using Reverse Transcription System kit (Promega Corp., USA). Next, suitable primers for the above-mentioned genes were prepared according to the operation manual provided by FastStart universal SYBR (a registered trademark) Greenmaster PCR KIT agent (Roche Diagnostics K. K., Germany).
Real time PCR system 7700HT (Applied Biosytems, USA) was used for the determination to carry out the real time PCR by using the cDNAs as templates. Each reaction conditions were set according to the manual of the PCR KIT. Quantitative analysis of the RNA amount was carried out based on comparative Ct method by using glyceraldehyde 3-phosphate dehydrogenase (GAPDH) as the standard reference.

The No200 administration group showed significantly high expression levels of GLUT4 and Adiponectin against the vehicle administration group (p <0.01). Between the vehicle administration group and P30 administration group, there were no significant differences. This showed that nobiletin has gene expression promotion effect of GLUT4, which is not shown by insulin resistance improving agent, Pioglitazone, in the white adipose tissue of the diabetes model mice. Alternatively, it showed that nobiletin has adiponectin secretagogue action through the promotion of adiponectin gene expression.
The expression levels of PEPCK, gluconeogenesis gene, were significantly low in both of the No200 administration group and the P30 administration group against that of the vehicle administration group (p <0.05). The G6Pase expression levels were similar (p <0.005). This showed that nobiletin has the inhibition effect of the gluconeogenesis expression-relating gene expression.

TNF-α gene expression level was significantly low in the No200 administration group against that of the vehicle administration group (p <0.005). However, that of the P30 administration group showed slight increase. Furthermore, MCP-1 gene and IL-6 gene expression levels were significantly low in both of the No200 administration group and P30 administration group against the vehicle administration group (p <0.005).
As mentioned above, it was demonstrated that nobiletin has the inhibition effect to the expression of bad adipokine genes such as TNF-α, MCP-1, IL-6 and the like in the diabetes model mice.

In the expression levels of PPARγ, there were no significant differences between the vehicle administration group and the P30 administration group. However, it was significantly increased in the No200 administration group (p <0.005). The expression level of aP2 was significantly increased in the P30 administration group compared to that of the vehicle administration group (p <0.005). However, it was significantly low in the No200 administration group compared to that of the P30 administration group (p <0.05). On the other hand, the expression level of the LPL was increased in the No200 administration group, and decreased in the P30 administration group. However, there were not significant differences. The Perilipin expression level was significantly increased in the No200 administration group compared to the vehicle administration group. However, there was no significant difference between that the P30 administration group and the vehicle administration group.
Accordingly, it was demonstrated that nobiletin enhances PPARγ expression level in the diabetes model mice, and has a particular effect to properly control blood lipid through the function of the adipose cells. Alternatively, it was demonstrated that SPE inhibits the lipid accumulation control-related gene and decrease the blood neutral fat concentration. Alternatively, it was demonstrated that nobiletin promotes the PPARγ expression, thereby promoting adiponectin secretion through adiponectin promotion.

Accordingly, it was demonstrated that nobiletin is different from thiazolidine type blood glucose hypotensive agent, and it enhances PPARγ function but it rarely causes the side effect, lipid biosynthesis increase.

### (5-3) Nobiletin ingestion and GLUT4 protein expression level

After finishing the experiment, the white adipose tissue and skeletal muscle were excised according to that described in the example 1, protein expression level of GLUT 4 were detected by using Western blotting method. The expression levels of GLUT4 protein were determined by using Na⁺/K⁺ ATPase α-1 protein as an internal reference. The results were shown in the table 11, Figs. 6B and 6C.

**[Table 11]**

| Tissue | Gene expression level (ratio) | | |
|---|---|---|---|
| | Vehicle | No200 | P30 |
| White adipose tissue | 1.00 | 1.75 | 2.24 |
| Skeletal muscle | 1.00 | 6.32 | 3.15 |

The expression level of the No200 administration group was increased 1.7 times higher than that of the vehicle administration group, and that of the P30 administration group was 2.2 times higher than it. By this, it was demonstrated that nobiletin has effect to promote GLUT4 protein expression in the white adipose tissue in the diabetes model mice.
As mentioned above, GLUT4 was highly expressed in the white adipose tissue when nobiletin was ingested. Therefore, it was demonstrated that there were no side effects such as increase of the blood neutral fat level and total cholesterol level, the weight gain of the white adipose tissue, the level change of blood insulin, weight gain of the liver, the body weight gain and change of the ingestion amount, even if the lipid synthesis associated with glucose intake enhancement has been activated.
Compared to the vehicle administration group, its expression amount of the No 200 administration group was about 6 times higher, and 3 times higher of the P30 administration group. This showed that nobiletin has excellent function to promote GLUT4 protein expression in the skeletal muscle of the diabetes model mice.

### Example 6

### 6. Effect of tangeretin to the obesity-induced mice by ingesting the high fat diet

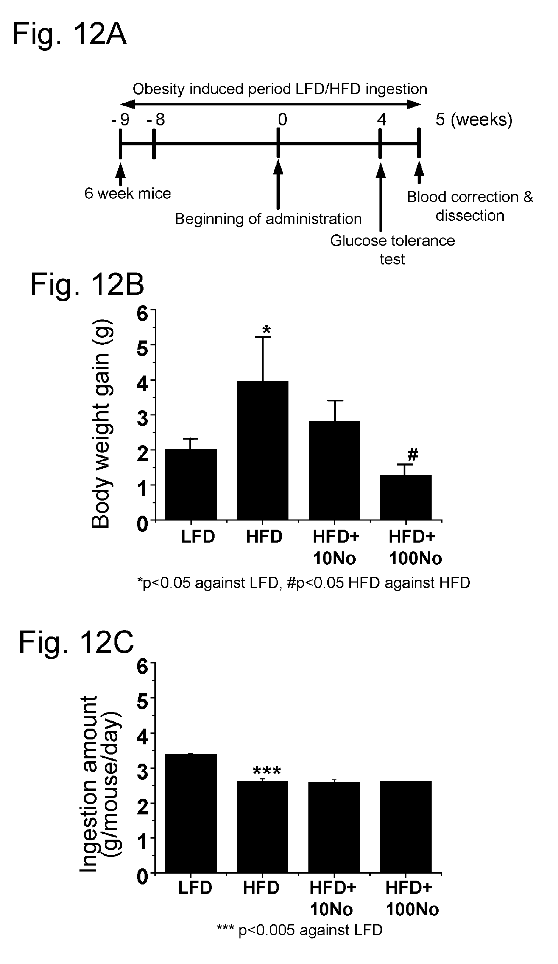

Six week age of C57BL/6 male mice were preliminary kept for 1 week (-9 to -8 week), and then classified into three groups. LED was given to one group, and HFD was given to other two groups, for 8 weeks (-8 to +5 week). From 8 weeks (15 week age) to 13 weeks, the following agents were given to each group for 5 weeks per os (hereinafter, they were referred to as LFD administration group and HFD administration group in the Example 6). The compositions of the LFD or HFD were the same as those in the Example 3.
To the LFD administration group (n= 8) and the HFD administration group (n= 11), 0.3% CMC aqueous solution was given at the amount of 0.01mL/g of the body weight. The group (n=11), to which tangeretin suspended in 0.3% CMC aqueous solution so as to administrate the amount of 0.01mL/g of body weight was given at the amount of 200mg/kg/day with HFD, was referred to as "T200 administration group; abbreviated as HFD + T200". The schedule of the feed ingestion and the agent administration of the above-mentioned 3 groups were schematically shown in Fig. 12A.

### (6-1) Effect of tangeretin ingestion to the body weight and ingestion amount

Results were shown in the table 12. The body weight gain after the termination of the experiment of the HFD administration group was significantly higher compared to that of the LFD administration group (p <0.005). Alternatively, there was no significant difference between that of the HFD administration group and the T200 administration group.
The ingestion amount of the mice in each group was measured twice a week. The ingestion amounts in 24 hours/3 mice /cage were measured and obtained as mean average of each group for 5 weeks. The ingestion amount of the HFD administration group was significantly low compared to that of the LFD administration group (p <0.005). There was no significant difference between the ingestion amount of the HFD administration group and the T200 administration group.

**[Table 12]**

| | Body weight gain and ingestion amount | | |
|---|---|---|---|
| Items measured | LFD | HFD | HFD+T200 |
| Body weight gain(g) | 1.53±0.32 | 4.04±0.42^{***} | 3.94±0.45 |
| Ingestion amount(g/day) | 3.27±0.19 | 2.50±0.26^{***} | 2.47±0.24 |

| | | | |
|---|---|---|---|
| ^{***}: p <0.005 | | | |

By this, it was indicated that the addition of 200mg/kg of tangeretin to the high fat diet did not affect the body weight gain and the ingestion amount. Since the ingestion amount was not changed, it was indicated that sole tangeretin has very small effect for ingestion behavior of metabolism.

### (6-2) Effects for the tissue weight by tangeretin ingestion

After the completion of the experiment, the mice in each group were dissected to excise organs and so forth to measure their weight. The white adipose tissue was excised from retroperitoneum and periphery of epididymis. Results were shown in the table 13.

**[Table 13]**

| | Tissue weight ( g/100g of body weight) | | |
|---|---|---|---|
| Tissue | LFD | HFD | HFD+T200 |
| White adipose tissue in total | 1.11±0.1 1 | 3.35±0.12^{***} | 3.38±0.16 |
| Retroperitoneum white adipose tissue | 0.81±0.0 6 | 2.35±0.11^{***} | 2.38±0.09 |
| Epididymis white adipose tissue | 0.33±0.0 4 | 0.99±0.07^{***} | 0.99±0.12 |
| Liver | 1.24±0.05 | 1.42±0.09 | 1.48±0.11 |
| Kidney | 0.30±0.00 | 0.35±0.02^{*} | 0.31±0.01 |

| | | | |
|---|---|---|---|
| ^{*}: p <0.05, ^{***}: p <0.005 | | | |

After the completion of the experiment, the weight of the white adipose tissue of the HFD administration group was significantly high compared to that of the LFD administration group (p <0.005). Furthermore, there were no significant changes of the weight between both of the white adipose tissue of the retroperitoneum and epididymis of the HFD administration group and those of the T200 administration group. Also, there was no significant difference between the total weights of the above-mentioned white adipose tissues in both groups.
There was no significant difference between the liver weight of the HFD administration group and the LFD administration group. It was the same between the HFD administration group and the HFD + T200 administration group. On the other hand, the kidney weight of the HFD administration group showed the significantly high compared to that of the LFD group (p <0.05). However, there was no significant difference between the kidney weight of the HFD administration group and the T200 administration group.
As mentioned above, it was demonstrated that 200mg/kg of tangeretin addition to the high fat diet did not affect the white adipose tissue weight and the liver weight. Further, it was demonstrated that the kidney weight gained by the high fat diet ingestion did not further increased.

### (6-3) Effect of tangeretin ingestion to the neutral fat and so forth

After the completion of the experiment, blood neutral fat level in fasting state was measured at the time points of beginning of the experiment, two and five weeks after the administration. Results were shown in the tables 14 and 15.
The blood neutral fat (TG) level was measured as the same as that employed in the example 1. The results were shown in the table 14. In the anytime points, that of the HFD administration group was significantly higher compared to that of the LFD administration group (p <0.005). However, there was no significant difference between that of the HFD administration group and that of the T200 administration group.

**[Table 14]**

| Time after administration (weeks) | Blood TG level (mg/dl) | | |
|---|---|---|---|
| | LFD | HFD | HFD+T200 |
| 0 | 76.93±8.40 | 108.67±5.93^{*} | 108.70±7.75 |
| 2 | 79.27±6.83 | 109.44±8.34^{*} | 107.89±9.46 |
| 5 | 67.64±3.66 | 103.21±12.61^{*} | 105.42±7.54 |

| | | | |
|---|---|---|---|
| ^{*}: p <0.05 | | | |

The total cholesterol (T-CHO) level in blood was measured as the same as that in the Example. The results were shown in the table 15. At the anytime points, that of the HFD ingestion group showed significantly high level compared to that of the LFD ingestion group (p <0.005). However, there was no significant difference between that of the HFD ingestion group and T200 administration group.
Accordingly, it was shown that the 200mg/kg of tangeretin addition to the feed did not affect the blood TG level and the total cholesterol level in blood when the high fat diet was ingested.

**[Table 7]**

| Time after administration (min.) | T-CHO level in blood (mg/dl) | | |
|---|---|---|---|
| | LFD | HFD | HFD+T200 |
| 0 | 96.80±7.87 | 190.89±4.66^{***} | 173.28±9.55 |
| 2 | 95.99±1.93 | 149.34±6.30^{***} | 144.77±8.05 |
| 5 | 90.36±5.22 | 172.49±12.30^{***} | 181.59±10.5 3 |

| | | | |
|---|---|---|---|
| ^{***}: p <0.005 | | | |

### (6-4) Effects of the tangeretin ingestion to the blood glucose value in fasting state

The blood glucose level was measured as the same as that of the example 1. The results described below were shown in the table 16. The table 16 shows that the effects by the tangeretin to the blood glucose level (mg/dl) on the high fat diet ingestion. In the any time points, that of the HFD administration group was significantly high compared to that of the LFD administration group (p <0.005). Furthermore, T200 administration group did not show the significant change to the HFD administration group 5 weeks from the beginning of the administration. However, it showed the significant low level at 2 weeks from the beginning of the administration (p <0.005). As mentioned above, it was shown that tangeretin has the inhibition effects of the blood glucose level in the fasting state when the high fat diet was ingested.

**[Table 16]**

| Time after administration (min.) | Blood glucose level (mg/dl) | | |
|---|---|---|---|
| | LFD | HFD | HFD+T200 |
| 0 | 97.89±2.48 | 136.80±3.93^{***} | 142.82±7.03 |
| 2 | 104.84±8.9 7 | 196.77±8.45^{***} | 164.79±9.82^{#} |
| 5 | 79.42±6. 91 | 143.91±6.97^{***} | 124.75±7.14 |

| | | | |
|---|---|---|---|
| ^{***}:p <0.005 ^{#}:p <0.05 | | | |

### (6-5) Effects of the tangeretin ingestion to the glucose tolerance

The oral glucose tolerance test (OGTT) was carried out as the same as that in the example 1. The results were shown in the table 17. At the any time points, the HFD administration group showed the significantly high blood glucose levels compared to those of the LFD administration group (p <0.005 to 0.005). However, there was no significant difference between the HFD administration group and the T200 administration group. By this, it was shown that 200mg/kg of tangeretin addition to the feed did not affect the glucose tolerance when the high fat diet was ingested.

**[Table 17]**

| Time after administration (min.) | Blood glucose level (mg/dl) | | |
|---|---|---|---|
| | LFD | HFD | HFD+T200 |
| 0 | 79.42±6.91 | 143.91±6.97^{***} | 124.75±7.14 |
| 15 | 302.92±15.15 | 354.00±16.66^{*} | 347.43±11.8 2 |
| 30 | 272.60±14.43 | 332.73±19.04^{*} | 309.73±17.8 8 |
| 60 | 216.22±13.17 | 284.23±17.32^{**} | 274.14±19.0 4 |
| 120 | 200.13±13.45 | 243.65±10.54^{*} | 265.19±15.82 |

| | | | |
|---|---|---|---|
| ^{*}: p <0.05, ^{**}: p <0.01 ^{***}: p <0.005 | | | |

### (6-6) Effects of tangeretin to the expression of glucose transporter and/or transcription factors

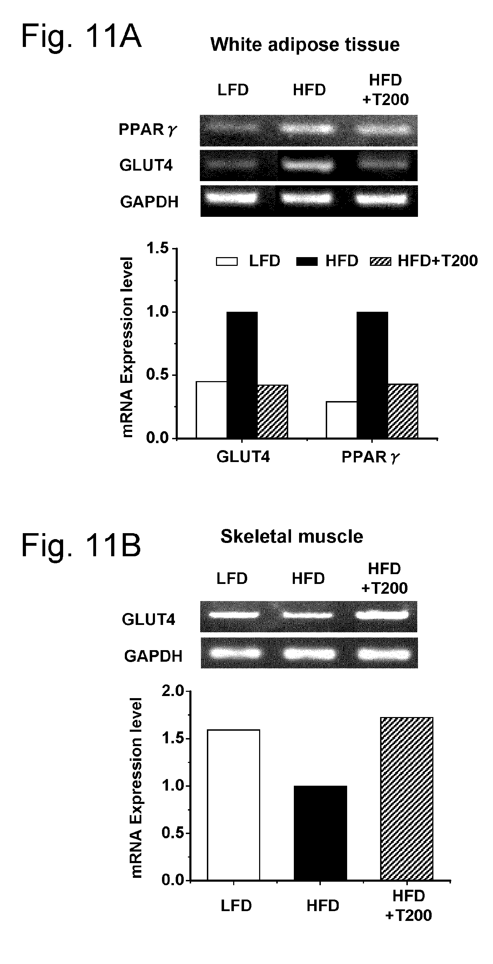

As the same as that in the Example 1, after the completion of the experiment, the white adipose tissue was excised to determine the both mRNA expression levels of GLUT4 gene mRNA and PPARγ gene by using the real time RT-PCR. Ratios of the expression levels when that of the HFD is 1 were shown in the table 18 and Figs. 11A and 11B.
When the mRNA expression levels of the GLUT4 mRNA and PPARγ in the white adipose tissue were compared, those of the HFD administration group were higher than those of the LFD administration group or those of the T200 administration group. On the other hand, the expression level of GLUT4 mRNA in the skeletal muscle of the HFD administration group was lower than that of the LFD administration group. However, that of the T200 administration group was higher than it.
As mentioned above, it was shown that tangeretin has the effect to inhibit the expression of the GLUT4 gene and PPARγ gene, which were enhanced by ingesting the high fat diet, in the white adipose tissue, and recover that of the GLUT gene, which was decreased by ingesting the high fat diet, in the skeletal muscle.

**[Table 18]**

| [Tissue] genes | Expression level (ratio) | | |
|---|---|---|---|
| | LFD | HFD | HFD+T200 |
| [W]GLUT4 | 0.45 | 1.00 | 0.42 |
| [W] PPARγ | 0.29 | 1.00 | 0.43 |
| [M]GLUT4 | 1.59 | 1.00 | 1.72 |

| | | | |
|---|---|---|---|
| W: white adipose tissue M: skeletal muscle | | | |

This indicates that tangeretin promotes the intake of blood glucose into the skeletal muscle when the high fat diet is ingested, and it leads to decrease the blood glucose level; and it prevents to intake of the blood glucose into the white adipose tissue to inhibit the enlargement of the adipose cells.
As mentioned above, it was considered that the expression levels of the GLUT4 genes in the white adipose tissue or skeletal muscle are effective indices for screening of insulin resistance improving agent, or prophylaxis and/or treatment agent for diabetes or life style disease.

### Example 7

### 7. Effect of nobiletin to the obesity-induced mice by the high fat diet ingestion

Six weeks age of male C57BL/6 mice were preliminary reared for 1 week (-9 to -8 week); then classified into 4 groups (n= 9 in each group). LFD was given to one group and HFD was given to other 3 groups for 8 weeks (-8 to +5 week). From 8 weeks (15 week age) to 13 weeks, each group was given the following agents per os for 5 weeks in everyday. In below, the LFD administration group was defined that they ingested LFD; the HFD administration group is defined that they ingested the HFD without nobiletin. The compositions of the LFD and HFD are the same as those used in the Example 3.
To both of the LFD administration group and the HFD administration group, 0.3% CMC aqueous solution was given at the amount of 0.01mL/g body weight. Hereinafter, in the example 7, addition to the HFD ingestion, nobiletin, which was suspended in 0.3 % CMC aqueous solution so as to be administrated at the amount of 0.01mL/g body weight. The group to which nobiletin was given at the amount of 10mg/kg/day was referred to as "No10 administration group; the abbreviated as HFD + No10" and another group to which nobiletin was given at the amount of 100mg/kg/day was referred to as "No100 administration group; abbreviated as HFD + No100". The schedule of the feed ingestion and administration of the agent for the above-mentioned 4 groups were schematically shown in Fig. 12A.

### (7-1) Effect of nobiletin to the body weight and the ingestion amount of the obesity-induced mice

Weight gain of each group was shown in the table 19 and Fig. 12B. In the table, [Final gain] means gained amount during 0 to +5 weeks. After the obesity induction by the high fat diet ingestion, the HFD administration group showed significantly heavy weight compared to that of the LFD administration group (p <0.005), gained weight amount was also the same. The No10 administration group did not have significant change of its gained weight compared to the HFD ingested group, however, the No10 administration group showed the significantly low (p <0.05). By this, it was shown that nobiletin has the effect to suppress the body weight gain after the obesity induction by ingesting the high fat diet.

**[Table 19]**

| | Body weight (g) | | | |
|---|---|---|---|---|
| Time after administration (weeks) | LFD | HFD | HFD+No10 | HFD+No10 0 |
| 0 | 25.68±0.42 | 31.32±0.81^{***} | 31.07±0.78 | 31.21±0.60 |
| 1 | 26.09±0.47 | 31.41±0.84^{***} | 31.10±0.72 | 29.93±0.52 |
| 2 | 26.66±0.53 | 32.62±1.13^{***} | 32.10±0.85 | 30.61±0.60 |
| 3 | 26.91±0.5 5 | 33.54±1.24^{* **} | 33.01±1.01 | 31.62±0.62 ^{#} |
| 4 | 27.11±0.4 8 | 33.28±1.31^{* **} | 32.58±0.97 | 31.28±0.59 ^{#} |
| 5 | 27.36±0.4 3 | 34.10±1.38^{* **} | 33.14±1.02 ^{#} | 31.88±0.68 |
| [Final gain] | 1.68±0.45 | 2.78±1.25^{*} | 2.08±0.69 | 0.67±0.37 ^{#} |

| | | | | |
|---|---|---|---|---|
| ^{*}:p <0.05 ^{***}:p <0.005 ^{#}:p <0.05 | | | | |

The change of the ingestion amount during the experiment term was shown in the table 20 and Fig. 12C. The ingestion amount of each mouse was measured everyday from 1 to 8 day (0 week) in the beginning of the agent administration. The ingested amount in 24 hrs was measured per cage, and then the average ingestion amount (g) per mouse was calculated. The date of day 0 means that of the previous day of the administration beginning. After 2 weeks from the administration, it was measured twice a week. [Final average] means the average of 5 weeks.
The ingestion amount of the HFD administration group was significantly low in 0 week compared to that of the LFD administration group (p <0.005), and the final average of the HFD administration group was also significantly low compared to that of the LFD administration group (p <0.005).

**[Table 20]**

| | Ingestion amount (g) | | | |
|---|---|---|---|---|
| Time after administration (day) | LFD | HFD | HFD+No10 | HFD+No100 |
| 0 | 3.06±0.07 | 2.36±0.04 | 2.36±0.04 | 2.36±0.04 |
| 1 | 3.39±0.09 | 2.12±0.21^{***} | 2.28±0.13 | 1.57±0.09 |
| 2 | 3.12±0.24 | 2.02±0.13^{***} | 1.85±0.19 | 1.71±0.13 |
| 3 | 3.12±0.24 | 2.02±0.13^{***} | 1.85±0.19 | 1.71±0.13 |
| 4 | 298±01 7 | 2.41±0.05^{***} | 2.27±0.28 | 1.83±0.15 ^{#} |
| 5 | 2.76±0.25 | 176±0.13^{***} | 1.78±0.21 | 1.59±0.20 |
| 6 | 3.33±0.19 | 2.32±0.11^{***} | 2.36±0.14 | 2.04±0.09 |
| 7 | 3.60±0.17 | 2.91±0.12^{***} | 2.99±0.07 | 2.62±0.12 |
| 8 | 3.27±0.19 | 2.19±0.25 | 2.12±0.32 | 2.43±0.03 |
| [Final Average] | 3.37±0.05 | 2.61±0.08 | 2.58±0.1 | 2.61±0.08 |

| | | | | |
|---|---|---|---|---|
| ^{***}: p <0.005 ^{#}: p <0.05 | | | | |

Also, the ingestion amount at day 4 of the No100 administration group was significantly low compared to that of the HFD administration group (p <0.05). However, in other time points, there were no significant differences between that of the No10 administration group and that of the No100 administration group. Furthermore, in the final average, there were no significant differences among that of the HFD administration group, No10 administration group, and No100 administration group.
As mentioned above, it was shown that nobiletin suppressed the ingestion amount in early stage, but it did not affect the ingestion amount through the period of the administration, when 100mg/kg nobiletin was administrated per os to the obesity-induced mice by using the high fat diet. This indicates that reduction of the body weight gain was not mainly caused by the decrease of the ingestion amount (see Figs. 12B and 12C).

### (7-2) Effect of nobiletin ingestion after obesity induction with the high fat diet to organs

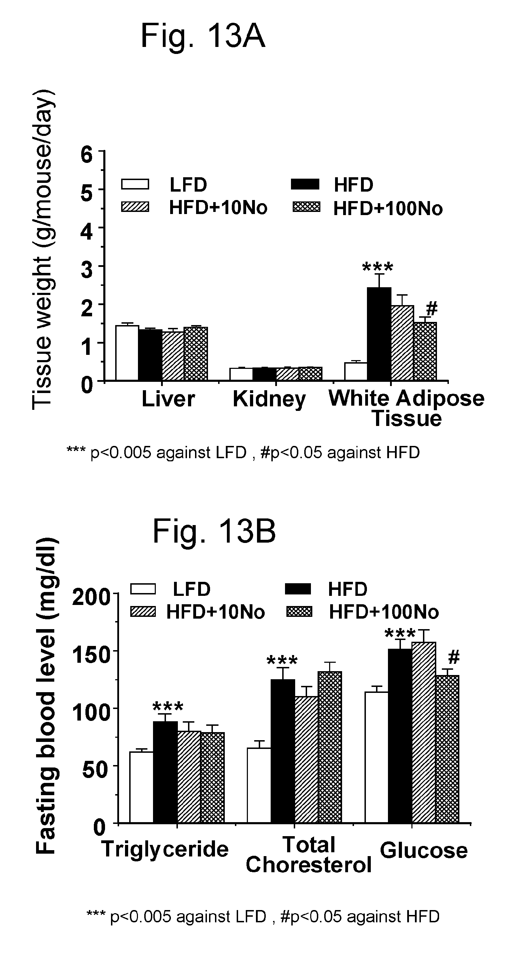

After the completion of the experiment, the mice are dissected to excised organs shown in the table 21, and then weighed. The results were shown in the table 21 and Fig. 13A.
Any group did not show the significant difference in the weight of liver and kidney. However, the weight of the adipose tissue of the HFD administration group was significantly high compared to that of the LFD ingestion group (p <0.005). However, that of the No100 administration group was significantly low compared to that of the HFD administration group (p <0.05).
As mentioned above, it was shown that 10 to 100mg/kg of nobiletin addition to the feed did not affect the weight of liver and kidney; however, 100mg/kg of it prevents the increase of the white adipose cells.

**[Table 21]**

| Tissue Weight (g) | Tissue Weight (g/ 100g of body weight) | | | |
|---|---|---|---|---|
| | LFD | HFD | HFD+No10 | HFD+No100 |
| Liver | 1.44±0.07 | 1.33±0.04 | 1.28±0.08 | 1.40±0.04 |
| Kidney | 0.33±0.02 | 0.34±0.01 | 0.34±0.02 | 0.35±0.01 |
| White adipose tissue | 0.47±0.06 | 2.42±0.37^{***} | 1.96±0.28 | 1.52±0.15 ^{#} |
| [Periphery of genitats] | 0.30±0.03 | 1.49±0.23^{***} | 1.23±0.19 | 0.92±0.09 ^{#} |
| [Periphery of kidney] | 0.07±0.02 | 0.57±0.09^{***} | 0.47±0.07 ^{#} | 0.36±0.03 |
| [Periphery of intestine] | 0.11±0.02 | 0.36±0.06^{***} | 0.27±0.04 | 0.20±0.02 ^{#} |

| | | | | |
|---|---|---|---|---|
| ^{***}: p <0.005 ^{#}: p <0.05 | | | | |

### (7-3) Effect of nobiletin ingestion to the blood lipid level after the obesity induction by ingesting the high fat diet

After the completion of the experiment, the levels of the blood neutral fat, total cholesterol, glucose, and adiponectin in the fating state, and those of the blood neutral fat, total cholesterol, and glucose in satiety state were determined as the same as those in the Example 1. In the table 22, relative adiponectin level means the ratio of specific level of the blood adiponectin to the total weight of the white adipose tissue.
The results were shown in the table 22. The results of the blood neutral fat (triglyceride), blood total cholesterol, and blood glucose levels were shown in Fig. 13B. The blood levels of the neutral fat and the total cholesterol both of the LFD ingestion group and the HFD ingestion group were remarkably low compared to those of the example 3 (3-3). It seems that these differences are derived from age of the mice and the period of the obesity induction.

**[Table 21]**

| Parameters | Blood lipid level Blood | | | |
|---|---|---|---|---|
| | LFD | HFD | HFD+No10 | HFD+No100 |
| Fasting state | | | | |
| Glucose | 113.99±5.41 | 151.19±8.67^{***} | 157.47±10.54 | 128.49±5.59 |
| TG | 62.07±2.66 | 88.03±7.15^{***} | 79.99±8.04 | 78.69±6.57 |
| T-CHO | 65.29±6.34 | 124.51±10.99^{* **} | 109.88±9.09 | 131.55±8.63 |
| Adipo *1 | 5.48±0.21 | 7.18±0.83 | 7.19±0.82 | 6.96±0.99 |
| Adipo *2 | 12.4±1.62 | 2.93±0.30^{***} | 3.87±0.86 | 5.34±0.97^{#} |
| Satiety state | | | | |
| Glucose | 225.59±16.4 5 | 238.42±14.25 | 224±24.67 | 224.76±21.88 ^{#} |
| TG | 175.60±30.69 | 138.50±13.18 | 141.05±12.25 | 100.80±14.95 |
| T-CHO | 95.68±5.99 | 153.23±15.26^{***} | 147.47±10.40 | 169.24±6.03 |

| | | | | |
|---|---|---|---|---|
| Except *1, unit is mg/dl, *1 adiponectin: unit is µg/mL, *2 Relative level of adiponectin: unit is µg/mL/g,^{***}: p <0.005 ^{#}: p <0.05 | | | | |

The blood glucose level of the HFD administration group in the fasting state was significantly high compared to that of the LFD administration group (p <0.005). There was no significant difference between the HFD administration group and the No10 administration group; however, there was significant difference between it and the No100 administration group (p <0.005). Note that there were no significant differences of the blood glucose level among each group in the satiety state.
In the blood TG level in the fasting state, the HFD administration group was significantly high compared to that of the LFD ingestion group (p <0.005). However, there was no significant difference between that of the No10 administration group and that of the No100 administration group. Note that there were no significant differences of the blood TG level among each group in the satiety state.
The T-CHO levels in blood of the HFD administration group was significantly high compared to those of the LFD administration group in both of the fasting and satiety state (p <0.005). There was no significant difference of T-CHO levels in blood in either fasting or satiety state among the HFD ingestion group and the No10 administration group and the No100 administration group.

Accordingly, it was shown that the 0 to 100mg/kg of nobiletin administration prevents the increase of the blood glucose level after the obesity induction by ingesting the high fat diet in the fasting state. However, it does not affect the blood glucose level when it was increased by the ingestion of feed. This shows that nobiletin does not suppress a digestion process from starch or polysaccharide to glucose in gastrointestinal system, or absorptive process of glucose, as a result, the blood glucose level was decreased. It showed that nobiletin enhances to absorb the glucose into any organs except the adipose tissue, liver and kidney, thereby decreasing the blood glucose level. Also, it showed that nobiletin did not affect blood TG level and T-CHO level after the obesity induction by ingesting the high fat diet.

In the HFD administration group, the blood adiponectin level after the obesity induction by ingesting the high fat diet showed a trend to increase, however there was no significant difference. Also, there was no significant difference among the HFD administration group, No10 administration group or No100 administration group.
Based on the levels measured, relative level of adiponectin was obtained as the specific level of blood adiponectin to the total weight of the white adipose tissue. In the HFD administration group, the blood adiponectin level was significantly decreased compared to that of the LFD administration group. Also, there was no significant difference between the HFD administration group and the No10 administration group. However, it showed significantly high in the No100 administration group.
The blood adiponectin level after the obesity induction by digesting the high fat diet ingestion was not changed. However, since the specific level of blood adiponectin was increased, the adiponectin production amount per unit weight of the white adipose tissue was increased by administration of 10 to 100mg/kg of nobiletin.
As mentioned above, when the obesity was induced by ingesting the high fat diet, it brings enlargement of the adipose cells causing insulin resistance; this leads the decrease of the adiponectin production amount per unit weight of the white adipose tissue; and nobiletin has recovery action to the decreased adiponectin production amount. Furthermore, it was shown that nobiletin enhances the expression of blood adiponectin to 2 to 3 timed higher, as shown in Patent Document 3 and the Example 5, was limited to the case at the onset of diabetes.

### (7-4) Effect of nobiletin to the glucose tolerance after the obesity induction by ingesting the high fat diet

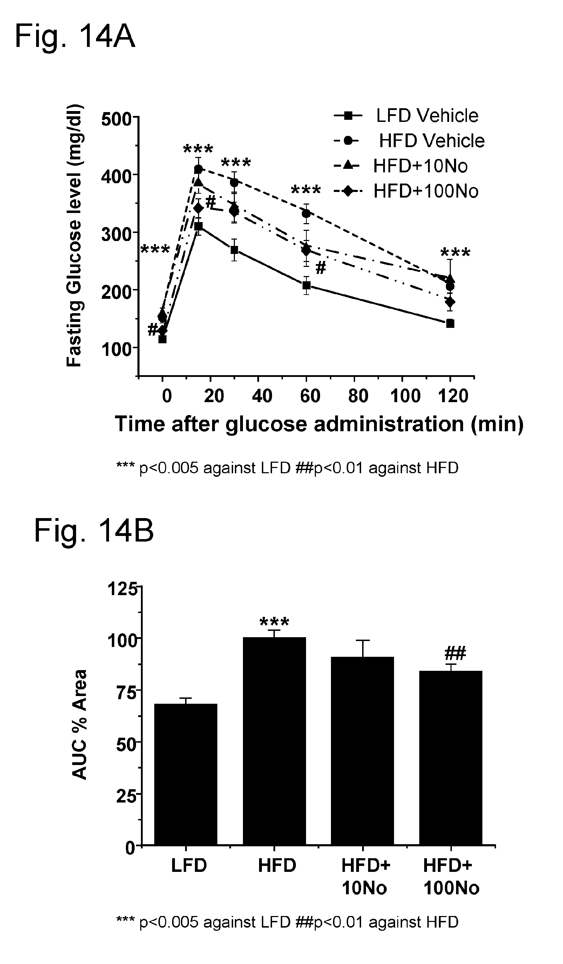

The sugar tolerance test was carried out as the same as those done in the example 2 (n= 9). Results were shown in the table 23, Figs. 14A and 14B.

**[Table 23]**

| Time after administration (minute) | Blood glucose level (mg/dl) | | | |
|---|---|---|---|---|
| | LFD | HFD | HFD+No10 | HFD+No100 |
| 0 | 113.99±5.41 | 151.19±8.67^{***} | 157.47±10.54 | 128.49±5.59 |
| 15 | 309.85±15.0 8 | 408.96±20.63^{* **} | 384.94±17.6 6 | 340.91±16.88 ^{#} |
| 30 | 269 ±19.16 | 385.77±19.12^{***} | 342.5±27.08 | 334.84±16.98 |
| 60 | 207.37±15.6 8 | 331.66±16.95^{* **} | 271.76±31.6 4 | 267.27±18.22 ^{#} |
| 120 | 141.25±7.15 | 205.57±11.09^{***} | 217.1±35.92 | 178.45±15.37 |

| | | | | |
|---|---|---|---|---|
| ^{***}: p <0.005 ^{#}: p <0.05 AUC: Integral value of the blood glucose concentration curve from time 0 to 120 minutes is shown as percentage (%), when that of the HFD is 100. | | | | |

At all of the time points, immediately after the glucose administration, 15 minute, 30 minute, 60 minute, 120 minutes, the blood glucose level was increased in the HFD administration group compared to that of the LFD administration group (all of p <0.05). There was no significant difference between the HFD administration group and the No10 administration group. On the other hand, between the HFD administration group and the No100 administration group, the blood glucose levels were respectively significantly low at 15 minutes and 60 minute (all of p <0.05).
Furthermore, in comparison of area under the curve (AUC) of the group, the HFD administration group showed significant increase than + LFD administration group (p <0.005). There was no significant difference between that of the HFD administration group and that of the No10 administration group. However, there was significant difference between that of the HFD ingestion group and that of the No100 administration group (p <0.05).
Accordingly, it was showed that 10 to 100mg/kg of nobiletin administration improves the glucose tolerance after the obesity induction by ingesting the high fat diet.

### (7-5) Effect of the nobiletin ingestion after the obesity induction by ingesting the high fat diet to size of the adipose cells

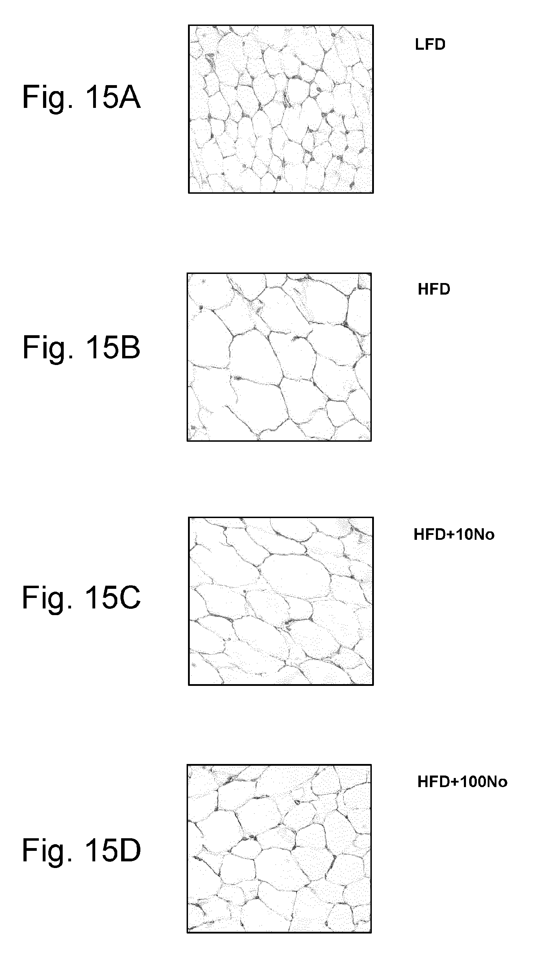

After the completion of the experiment, the adipose tissue of mice epididymis was excised to prepare sections to stain by using both of hematoxylin and eosin (n= 9). These sections were observes under the microscope with 200-fold magnification to compare cell seizes in the field of photomicroscope vision. The results were shown in Figs. 15A to 15D.

In the HFD administration group (Fig. 15B), the adipose cell size after the experiment completion became larger compared to those in the LFD administration group (Fig. 15A). There was not large difference between that of the HFD ingestion group and the No10 administration group (Fig. 15C). However, in the No100 administration group (Fig. 15D); the size of the adipose cells became smaller.
As mentioned above, it was shown that nobiletin has the effect to shrink the adipose cells of the white adipose tissue after obesity induction by ingesting the high fat diet. This result was not observed in the diabetes model mice used in the example 2. Namely, it was shown that 200mg/kg of nobiletin administration fails to shrink the adipose cells enlarged when onset of diabetes of the white adipose tissue; however, 10 to 100mg/kg of nobiletin enables to shrink the cells in obesity by ingesting the high fat diet, that is, before diabetes onset.
These observation results show that nobiletin promotes downsizing the adipose cells in the white adipose tissue to improve insulin resistance before the diabetes onset though the proliferation of the adipose cells to divide enlarged adipocytes or to replace the enlarged adipocytes with adipose cells newly proliferated.

### (7-6) Nobiletin ingestion after the obesity induction by ingesting the high fat diet and the gene expression in the white adipose tissue

The expression of mouse lipid biosynthesis-related gene and other gene shown in the following table 24 after obesity induction by ingesting the high fat diet were determined as mRNA levels to study the effects of nobiletin to these gene expression (n= 9). The expression levels of each gene were shown in the ratio when those of the HFD were 1. The results were shown in the table 24.

**[Table 24]**

| Genes | Expression levels of each gene | | | |
|---|---|---|---|---|
| | LFD | HFD | HFD+No10 | HFD+No100 |
| SREBP1-c | 1.34±0.39 | 1.00±0.01 | 2.15±0.21 | 1.53±0.20 |
| SCD1 | 1.32±0.08 | 1.00±0.06^{*} | 2.30±0.06^{###} | 2.95±0.17^{###} |
| FAS | 3.64±0.05 | 1.00±0.06^{***} | 2.78±0.06^{###} | 2.50±0.03^{###} |
| ACC1 | 4.24±0.11 | 1.00±0_{.}03^{***} | 2.06±0.05^{###} | 1.69±0.07^{###} |
| DGAT1 | 0.89±0.06 | 1.00±0.08 | 2.17±0.19^{##} | 1.80±0.14^{##} |
| GLUT4 | 1.35±0.11 | 1.00±0.09^{*} | 1.34±0.09 | 1.35±0.07 |
| Adiponectin | 0.60±0.01 | 1.00±0.08^{*} | 1.97±0.09 | 2.02±0.08 |
| TNF-α | 0.43±0.09 | 1.00±0.11^{*} | 1.06±0.12 | 0.58±0.14^{#} |
| MCP-1 | 0_{.}08±0_{.}00 | 1.00±0_{.}05^{***} | 0.44±0.02^{##} | 0.35±0.01^{##} |
| PPARy | 0.74±0.04 | 1.00±0.05^{*} | 1.61±0.11^{##} | 1.36±0.05^{##} |

| | | | | |
|---|---|---|---|---|
| ^{*}: p <0.05 ^{***}: p <0.005 ^{#}: p <0.05 ^{##}: p <0.01 ^{###}: p <0.005 | | | | |

In the HFD administration group, the expression levels of mRNA of SCD1, FAS, ACC1, and GLUT4 were significantly low compared to those in the LFD administration group(p <0.05 in SCD1 and GLUT4, p <0.005 in FAS and ACC1). Also, in the HFD administration group, the expression levels of mRNA of adiponectin, TNF-α, MCP-1 and PPARγ were significantly high compared to the LFD administration group (<0.05 in adiponectin, TNF-α and PPARγ; p <0.005 in MCP-1).
In the HFD + No10 administration group and + No100 administration group, the expression levels of mRNA of SCD1, FAS, ACC1, DAGT1, PPARγ, and SREBP1-c were significantly high compared to those in the HFD ingestion group (p <0.01 in DAGT1and PPARγ; p< 0.005 in SCD1, FAS, ACC1, SREBP1-c). There was no significant difference of the expression level of TNF-α mRNA between those of the HFD administration group and the No10 administration group; however, that of the No100 administration group was significantly low.
As mentioned above, it was found that nobiletin affects the gene expression of the white adipose tissue.

Tangeretin has the effect to decrease the fasting blood glucose level (blood glucose level); however, it is not always effective to impaired glucose tolerance. Nobiletin affects to the fasting blood glucose level and the impaired glucose tolerance. It was found that the composition composed of 50 wt % of nobiletin and 50 wt% of tangeretin enables to inhibit hyperlipidemia.

### Industrial Applicability

The present invention is useful in the fields of production and development of a pharmaceutical preparation, functional food, healthy food and the like.

## Claims

1. A method for screening a treatment method for metabolic syndrome accompanying visceral fat, hyperglycemia and hyperlipidemia, comprising the steps (1) to (3) of:
(1) extracting total mRNA fraction from skeletal muscle excised from a non-human mammal animal selected from the group consisting of a test substance administration group and a test substance non-administration group, wherein the animal of the test substance administration group is given the test substance at an predetermined amount in predetermined term and that of the test substance non-administration group is not given the test substance;
(2) determining an expression amount of mRNA that codes glucose transporter gene included in the respective total RNA fraction extracted in the extraction step by using a predetermined method; and
(3) deciding an effect to the metabolic syndrome by using comparison analysis for body weight gain amount and ingestion amount, and the expression amount of RNA of the glucose transporter gene in the predetermined period between the test substance administration group and the test substance non-administration group,

2. The method according to the claim 1, wherein the glucose transporter gene is mouse GLUT 4 and homolog thereof.

3. The method according to the claims 1 or 2, wherein comprising the steps of:
synthesizing cDNA from RNA that codes the glucose transporter gene by using a reverse transcriptase to amplify the cDNA with a specific primer for the glucose transporter gene,
determining the amplified products by using a detection method that matches to a regent selected from the group consisting of fluorescently-labeled nucleic acid, isotope-labeled nucleic acid, and nucleic acid staining; and
determining the expression amount of RNA that codes glucose transporter gene.

4. A composition for treating the metabolic syndrome accompanying visceral fat type obesity, hyperglycemia and hyperlipidemia in precritical stage, comprising a peel extract fraction of Mikan-ku (Acrumen (VII) section) citrus that includes 70 to 100 wt % of nobiletin, and administering a dose of 5 to 200 mg/kg of the fraction convert to dry weight of the peel fraction having following functions:
(1) a function to reduce a body weight gain without any affection for ingestion amount;
(2) a function to promote expression of protein of a mouse glucose transporter gene, GLUT 4 and a homolog thereof, in a skeletal muscle tissue or a skeletal muscle cell;
(3) a function to promote to shrink an enlarged white adipose tissue located periphery of viscera; and
(4) a function to decrease blood glucose level without any affection to blood triglyceride.

5. The composition for treating the metabolic syndrome according to the claim 4, wherein the Mikan-ku (Acrumen (VII) section) citrus is flat lemon (Shiikuwasha; C. depressa), wherein nobiletin content in the peel extract fraction is 95 to 100 wt % in dry weight, the dose is 10 to 100 mg/kg per day.

6. A composition for prophylaxis and/or treatment of the metabolic syndrome accompanying visceral fat type obesity, hyperglycemia and hyperlipidemia, comprising peel extract fraction of Mikan-ku (Acrumen (VII) section) citrus that includes 45 to 55 wt % of nobiletin and 45 to 55 % of tangeretin, and administering at a dose of 0.5 to 8.0 g/body/day of the fraction of the peel fraction in dry weight equivalent having following functions:
(1) a function to reduce a body weight gain without any affection for ingestion amount;
(2) a function to promote expression of mRNA or protein of a mouse glucose transporter gene, GLUT 4 and a homolog thereof, in a skeletal muscle tissue or a skeletal muscle cell;
(3) a function to promote to shrink an enlarged white adipose tissue located periphery of viscera; and
(4) a function to decrease blood triglyceride concentration without any affection to blood glucose.

7. The composition for prophylaxis and/or treatment of the metabolic syndrome according to the claim 6, wherein the Mikan-ku (Acrumen (VII) section) citrus is flat lemon: Citrus depressa and the dose is 1.0 to 6.0 g/body/day.

8. A functional food comprising the composition for treating the metabolic syndrome of any one of claims 4 to 7.

9. A healthy food comprising the composition for treating the metabolic syndrome of any one of claims 4 to 7.

10. A pharmaceutical preparation comprising the composition for treating the metabolic syndrome of any one of claims 4 to 7.
